(19) Europäisches Patentamt
European Patent Office
Office européen des brevets

(11) **EP 4 289 913 A1**

(12) **EUROPEAN PATENT APPLICATION**
published in accordance with Art. 153(4) EPC

(43) Date of publication:
**13.12.2023 Bulletin 2023/50**

(21) Application number: **22749943.1**

(22) Date of filing: **27.01.2022**

(51) International Patent Classification (IPC):
***C09J 11/06*** *(2006.01)* ***C08K 5/3445*** *(2006.01)*
***C08K 5/3462*** *(2006.01)* ***C08K 5/45*** *(2006.01)*
***C09J 7/22*** *(2018.01)*

(52) Cooperative Patent Classification (CPC):
**C08K 5/3445; C08K 5/3462; C08K 5/45; C09J 7/22; C09J 11/06**

(86) International application number:
**PCT/KR2022/001436**

(87) International publication number:
**WO 2022/169192 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.02.2021 KR 20210014649**
**09.06.2021 KR 20210074736**

(71) Applicant: **Lg Chem, Ltd.**
**Seoul 07336 (KR)**

(72) Inventors:
- **SON, Seonkyoung**
  **Daejeon 34122 (KR)**
- **LE, Duy Hieu**
  **Daejeon 34122 (KR)**
- **AN, Junhyun**
  **Daejeon 34122 (KR)**
- **LEE, Hoyong**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **PYRAZOLE DERIVATIVE COMPOUND, ADHESIVE COMPOSITION COMPRISING SAME, AND ADHESIVE FILM COMPRISING SAME**

(57) The present invention relates to a pyrazole derivative compound, an adhesive composition including the same and an adhesive film including the same.

[Figure 1]

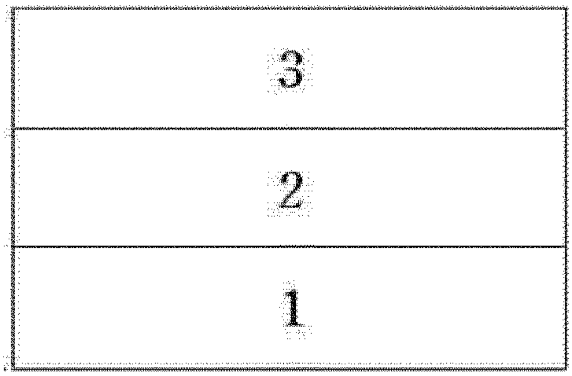


EP 4 289 913 A1

## Description

[Technical Field]

The present application claims priority to and the benefit of Korean Patent Application Nos. 10-2021-0014649 and 10-2021-0074736 filed in the Korean Intellectual Property Office on February 2, 2021 and June 9, 2021, respectively, the entire contents of which are incorporated herein by reference.

The present invention relates to a pyrazole derivative compound, an adhesive composition including the same and an adhesive film including the same.

[Background Art]

A display device included in an electronic device typically implements an image with a combination of lights having a specific wavelength of R (Red), G (Green), and B (Blue). In this case, in the case of indoors, the visibility of the display device is good because there is no external light having high energy. However, in the case of outdoors, strong light reflection occurs on the surface of the device, electrodes in the device, and the like due to external light having high energy such as sunlight, and accordingly, the amount of light emitted from the display is reduced, so that there is a problem in that the contrast ratio and visibility of the display are significantly reduced.

In order to solve the problems of a reduction in the contrast ratio and a reduction in visibility due to the reflection of external light in the display, there has been an attempt to reduce the reflected light by designing a display so as to include a region that absorbs external light in the display. For example, in the case of an organic light emitting device (OLED), a polarizing plate is included in the device in order to reduce the reflected light due to the aforementioned external light. The polarizing plate included in the electronic device improves surface reflection, electrode reflection, and image emission brightness by adjusting the amount of external light absorbed.

However, when a polarizing plate is used for an electronic device to adjust the amount of external light, there are problems in that the hue of light emitting color cannot be flexibly adjusted, the material cost is increased, and the device structure cannot be flexibly designed.

[Citation List]

(Patent Document 1) Japanese Patent Application Laid-Open No. 2012-211305

[Detailed Description of the Invention]

[Technical Problem]

An object of the present invention is to provide a compound of Chemical Formula H of the present application.

Another object of the present invention is to provide an adhesive composition including the compound of Chemical Formula H of the present application and/or an adhesive film including the above-described adhesive composition.

Still another object of the present invention is to provide an electronic device including the above-described adhesive film.

[Technical Solution]

An exemplary embodiment of the present invention provides a compound represented by the following Chemical Formula H.

[Chemical Formula H]

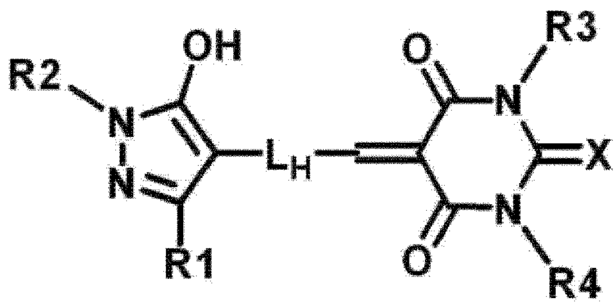

In Chemical Formula H,

X is O or S,
$L_H$ is a direct bond; or a substituted or unsubstituted alkylene group,
R1 is -C(=O)ORa; a substituted or unsubstituted alkyl group; a substituted or unsubstituted cycloalkyl group; a substituted or unsubstituted aryl group; or a substituted or unsubstituted heterocyclic group,
R2 is -C(=O)Rb; a substituted or unsubstituted alkyl group; a substituted or unsubstituted cycloalkyl group; a substituted or unsubstituted aryl group; or a substituted or unsubstituted heterocyclic group,
Ra is hydrogen; deuterium; or a substituted or unsubstituted alkyl group,
Rb is hydrogen; deuterium; a hydroxyl group; a substituted or unsubstituted alkyl group; a substituted or unsubstituted aryl group; or a substituted or unsubstituted heterocyclic group, and
R3 and R4 are the same as or different from each other, and are each independently a substituted or unsubstituted cycloalkyl group; or a substituted or unsubstituted aryl group.

According to another exemplary embodiment of the present invention, an adhesive composition including the above-described compound is provided.
Still another exemplary embodiment of the present invention provides an adhesive film including the above-described adhesive composition.
Yet another exemplary embodiment of the present invention provides an electronic device including the above-described adhesive film.

[Advantageous Effects]

The compound according to an exemplary embodiment of the present invention, the adhesive composition including the same, and/or the adhesive film including the same strongly absorb(s) light in a specific wavelength region to improve the light blocking efficiency.
The compound according to an exemplary embodiment of the present invention, the adhesive composition including the same, and/or the adhesive film including the same have a reduced reflectance to external light.
The compound according to an exemplary embodiment of the present invention, the adhesive composition including the same, and/or the adhesive film including the same have improved light fastness, heat resistance, moisture resistance, solubility to solvent, and/or wavelength compatibility.

[Brief Description of Drawings]

FIG. 1 illustrates an adhesive film including the adhesive composition according to an exemplary embodiment of the present invention.
FIG. 2 illustrates the solution-phase UV-Vis spectra of Compound P3 according to an exemplary embodiment of the present invention and Comparative Compound Z2 used in Comparative Example 2.

[Explanation of Reference Numerals and Symbols]

1: Glass
2: Adhesive film
3: Binder resin film

[Best Mode]

Hereinafter, the present invention will be described in detail.

An exemplary embodiment of the present invention provides a compound represented by the following Chemical Formula H.

[Chemical Formula H]

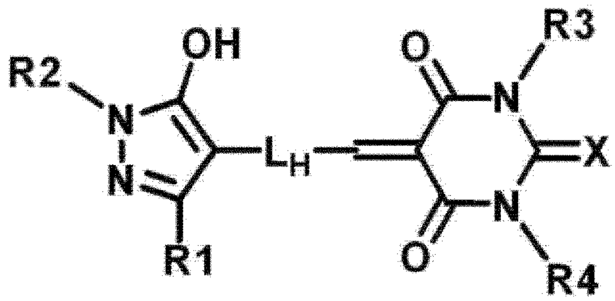

In Chemical Formula H,

X is O or S,
$L_H$ is a direct bond; or a substituted or unsubstituted alkylene group,
R1 is -C(=O)ORa; a substituted or unsubstituted alkyl group; a substituted or unsubstituted cycloalkyl group; a substituted or unsubstituted aryl group; or a substituted or unsubstituted heterocyclic group,
R2 is -C(=O)Rb; a substituted or unsubstituted alkyl group; a substituted or unsubstituted cycloalkyl group; a substituted or unsubstituted aryl group; or a substituted or unsubstituted heterocyclic group,
Ra is hydrogen; deuterium; or a substituted or unsubstituted alkyl group,
Rb is hydrogen; deuterium; a hydroxyl group; a substituted or unsubstituted alkyl group; a substituted or unsubstituted aryl group; or a substituted or unsubstituted heterocyclic group, and
R3 and R4 are the same as or different from each other, and are each independently a substituted or unsubstituted cycloalkyl group; or a substituted or unsubstituted aryl group.

A pyrazole derivative compound of Chemical Formula H according to an exemplary embodiment of the present invention may adjust the length of the molecule by having a bent structure because the pyrazole derivative is not directly bonded to a hetero ring, but is linked to the hetero ring via an alkylene group. Accordingly, the pyrazole derivative compound of Chemical Formula H of the present application has a resonance structure and a molecular structure arrangement capable of hydrogen bonding, and thus can secure a maximum absorption wavelength of 380 to 450 nm, so that there is an advantage in that the pyrazole derivative compound has improved wavelength compatibility and excellent light fastness, heat resistance or mosture resistance.

When one member (layer) is disposed "on" another member (layer) in the present invention, this includes not only a case where the one member (layer) is brought into contact with another member, but also a case where still another member (layer) is present between the two members (layers).

When one part "includes" one constituent element in the present invention, unless otherwise specifically described, this does not mean that another constituent element is excluded, but means that another constituent element may be further included.

In the present invention, the "layer" has a meaning compatible with a "film" usually used in the art, and means a coating covering a target region. The size of the "layer" is not limited, and the sizes of the respective "layers" may be the same as or different from one another. According to an exemplary embodiment, the size of the "layer" may be the same as that of the entire device, may correspond to the size of a specific functional region, and may also be as small as a single sub-pixel.

Unless otherwise defined in the present invention, all technical and scientific terms used in the present invention have the same meaning as commonly understood by one with ordinary skill in the art to which the present invention pertains. Although methods and materials similar to or equivalent to those described in the present invention may be

used in the practice or in the test of exemplary embodiments of the present invention, suitable methods and materials will be described below. All publications, patent applications, patents, and other references mentioned in the present invention are hereby incorporated by reference in their entireties, and in the case of conflict, the present invention, including definitions, will control unless a particular passage is mentioned. In addition, the materials, methods, and examples are illustrative only and are not intended to be limiting.

In the present invention, the term "combination thereof" included in the Markush type expression means a mixture or combination of one or more selected from the group consisting of constituent elements described in the Markush type expression, and means including one or more selected from the group consisting of the above-described constituent elements.

In the present invention,

" 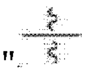 "

means a site to be linked.

Examples of the substituents in the present invention will be described below, but are not limited thereto.

In the present invention, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present invention, the term "substituted or unsubstituted" means being substituted with one or more substituents selected from the group consisting of deuterium; a halogen group; a nitrile group; an alkyl group; a cycloalkyl group; an amine group; an aryl group; and a heteroaryl group, being substituted with a substituent to which two or more substituents among the exemplified substituents are linked, or having no substituent.

In the present invention, the fact that two or more substituents are linked indicates that hydrogen of any one substituent is linked to another substituent. For example, an isopropyl group and a phenyl group may be linked to each other to become a substituent of

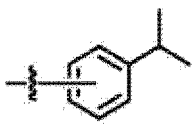

or

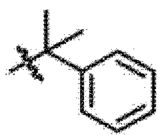

.

In the present invention, the fact that three substituents are linked to one another may include not only a case where (Substituent 1)-(Substituent 2)-(Substituent 3) are consecutively linked to one another, but also a case where (Substituent 2) and (Substituent 3) are linked to (Substituent 1). For example, two phenyl groups and an isopropyl group may be linked to each other to become a substituent of

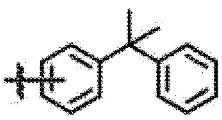

or

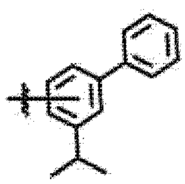

.

The same also applies to the case where four or more substituents are linked to one another.

In the present invention, "-$L_H$-=" is the same as "-$L_H$-C=C" because "-$L_H$-=" means any one of the carbons linked by a double bond is linked to $L_H$. As an example, when $L_H$ is a direct bond, "-$L_H$-=" is "-C=C". As another example, when $L_H$ is a methylene group, "-$L_H$-=" is "-C-C=C".

In the present invention, a halogen group is a fluoro group (-F), a chloro group (-Cl), a bromo group (-Br), or an iodo group (-I).

In the present invention, a nitrile group is -CN, coordinates to a metal, is easily converted between a coordinated state and a free non-coordinated state, and is a non-ionic functional group.

In the present invention, an alkyl group may be straight-chained or branched, and the number of carbon atoms

thereof is not particularly limited, but is preferably 1 to 30; 1 to 20; 1 to 10; or 1 to 5. Specific examples thereof include methyl, ethyl, propyl, n-propyl, isopropyl, butyl, n-butyl, isobutyl, t-butyl (tert-butyl), sec-butyl, 1-methylbutyl, 1-ethylbutyl, pentyl, n-pentyl, isopentyl, neopentyl, t-pentyl, hexyl, n-hexyl, 1-methylpentyl, 2-methylpentyl, 3,3-dimethylbutyl, 2-ethylbutyl, heptyl, n-heptyl, 1-methylhexyl, cyclopentylmethyl, cyclohexylmethyl, octyl, n-octyl, t-octyl, 1-methylheptyl, 2-ethylhexyl, 2-propylpentyl, n-nonyl, 2,2-dimethylheptyl, 1-ethylpropyl, 1,1-dimethylpropyl, isohexyl, 4-methylhexyl, 5-methylhexyl, and the like, but are not limited thereto. The alkyl group may include an alkyl group substituted with a halogen group. Specific examples thereof include a methyl group substituted with three fluoro groups, that is, a trifluoromethyl group, and the like, but are not limited thereto.

In the present invention, the above-described description on the alkyl group is applied to the alkylene group except for a divalent alkylene group.

In the present invention, a cycloalkyl group is not particularly limited, but the number of carbon atoms thereof is not particularly limited, but is preferably 3 to 60; 3 to 30; or 3 to 20. Specific examples of the cycloalkyl group include a cyclopropyl group, a cyclobutyl group, a cyclopentyl group, a cyclohexyl group, a cycloheptyl group, a cyclooctyl group, and the like, but are not limited thereto.

In the present invention, an aryl group means a monovalent aromatic hydrocarbon or a monovalent group of an aromatic hydrocarbon derivative. In the present invention, an aromatic hydrocarbon means a compound in which pi electrons are completely conjugated and which contains a planar ring, and a group derived from an aromatic hydrocarbon means a structure in which an aromatic hydrocarbon or a cyclic aliphatic hydrocarbon is fused with an aromatic hydrocarbon. Further, in the present invention, an aryl group intends to include a monovalent group in which two or more aromatic hydrocarbons or derivatives of an aromatic hydrocarbon are linked to each other. The aryl group is not particularly limited, but preferably has 6 to 60 carbon atoms; 6 to 50 carbon atoms; 6 to 30 carbon atoms; 6 to 25 carbon atoms; 6 to 20 carbon atoms; 6 to 18 carbon atoms; 6 to 15 carbon atoms; 6 to 13 carbon atoms; or 6 to 12 carbon atoms, and may be a monocyclic aryl group or a polycyclic aryl group.

The monocyclic aryl group is not particularly limited, but preferably has 6 to 60 carbon atoms; 6 to 54 carbon atoms; 6 to 48 carbon atoms; 6 to 42 carbon atoms; 6 to 36 carbon atoms; 6 to 30 carbon atoms; 6 to 24 carbon atoms; 6 to 18 carbon atoms; or 6 to 12 carbon atoms, and may be specifically a phenyl group, a biphenyl group, a terphenyl group, and the like, but is not limited thereto.

The polycyclic aryl group is not particularly limited, but preferably has 6 to 60 carbon atoms; 6 to 45 carbon atoms; 6 to 30 carbon atoms; 6 to 25 carbon atoms; 6 to 22 carbon atoms; 6 to 20 carbon atoms; 6 to 18 carbon atoms; 6 to 16 carbon atoms; 6 to 15 carbon atoms; 6 to 14 carbon atoms; 6 to 13 carbon atoms; 6 to 12 carbon atoms; or 6 to 10 carbon atoms, and may be a naphthyl group, an anthracenyl group, a phenanthrenyl group, a pyrenyl group, a perylenyl group, a triphenylenyl group, a chrysenyl group, a fluorenyl group, and the like, but is not limited thereto.

In the present invention, a heteroaryl group means a monovalent aromatic hetero ring. Here, the aromatic hetero ring is a monovalent group of an aromatic ring or a derivative of the aromatic ring, and means a group including one or more selected from the group consisting of N, O, P, S, Si and Se as a heteroatom. The derivative of the aromatic ring all includes a structure in which an aromatic ring or an aliphatic ring is fused with an aromatic ring. Further, in the present specification, the heteroaryl group intends to include a monovalent group in which two or more aromatic rings including a heteroatom or two or more derivatives of an aromatic ring including a heteroatom are linked to each other. The number of carbon atoms of the heteroaryl group is 2 to 60; 2 to 50; 2 to 30; 2 to 20; 2 to 18; or 2 to 13. Examples of the heteroaryl group include a thiophene group, a furanyl group, a pyrrole group, an imidazole group, a thiazole group, an oxazole group, a pyridine group, a pyrimidine group, a triazine group, a triazole group, an acridine group, a pyridazine group, a pyrazine group, a quinoline group, a quinazoline group, a quinoxaline group, an isoquinoline group, an indole group, a carbazole group, a benzoxazole group, a benzimidazole group, a benzothiazole group, a benzocarbazole group, a benzothiophene group, a dibenzothiophene group, a benzofuran group, a phenanthrolinyl group, a dibenzofuran group, and the like, but are not limited thereto.

In the present invention, the heteroaryl group may be monocyclic or polycyclic.

In the present invention, the heterocyclic group is a monovalent group of an aliphatic ring, a derivative of the aliphatic ring, an aromatic ring or a derivative of the aromatic ring, and means a group including one or more selected from the group consisting of N, O, P, S, Si and Se as a heteroatom.

In the present invention, the aliphatic ring is not an aromatic but a hydrocarbon ring, and examples thereof include examples of the above-described cycloalkyl group, an adamantyl group, and the like.

In the present invention, the above-described content on the aryl group may be applied to an aromatic ring.

In the present invention, the above-described description on the heterocyclic group may be applied to a hetero ring except that the hetero ring is a divalent group.

In the present invention, the above-described description on the aryl group may be applied to an aromatic hydrocarbon ring except that the aromatic hydrocarbon ring is a divalent group.

In the present invention, the above-described description on the cycloalkyl group may be applied to an aliphatic hydrocarbon ring except that the aliphatic hydrocarbon ring is a divalent group.

In the present invention, the group in the parenthesis means a substituted group. For example, -N(Rk) means -N substituted with Rk. As another example, -C(= O)Rk means that -C and O are linked by a double bond and -C is linked to Rk. As still another example, -C(=O)ORk means that -C and any one O are linked by a double bond, -C is linked to the other O, and the other O is linked to Rk.

In the present invention, a solid content means a component except for a solvent in a composition. Specifically, the total weight of the solid content means the sum of total weights of components except for a solvent in a photosensitive resin composition. The basis of wt% based on the solid content and a solid content of each component may be measured by a general analysis means used in the art, such as liquid chromatography or gas chromatography.

According to an exemplary embodiment of the present invention, Chemical Formula H is represented by the following Chemical Formula H-1.

[Chemical Formula H-1]

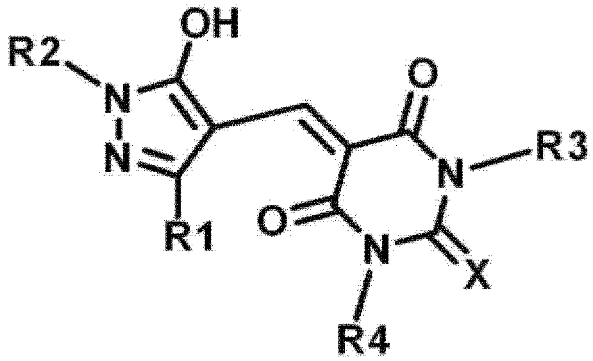

In Chemical Formula H-1,

X and R1 to R4 are the same as those defined in Chemical Formula H.

According to an exemplary embodiment of the present invention, X is O or S.

According to an exemplary embodiment of the present invention, X is O.

According to an exemplary embodiment of the present invention, X is S.

According to an exemplary embodiment of the present invention, $L_H$ is a direct bond; or a substituted or unsubstituted alkylene group.

According to an exemplary embodiment of the present invention, $L_H$ is a direct bond; or a substituted or unsubstituted alkylene group having 1 to 10 carbon atoms.

According to an exemplary embodiment of the present invention, $L_H$ is a direct bond.

According to an exemplary embodiment of the present invention, R1 is -C(=O)ORa; a substituted or unsubstituted alkyl group; a substituted or unsubstituted cycloalkyl group; a substituted or unsubstituted aryl group; or a substituted or unsubstituted heterocyclic group, and Ra is hydrogen; deuterium; or a substituted or unsubstituted alkyl group.

According to an exemplary embodiment of the present invention, R1 is a substituted or unsubstituted alkyl group; a substituted or unsubstituted cycloalkyl group; or a substituted or unsubstituted aryl group.

According to an exemplary embodiment of the present invention, R1 is an alkyl group; a cycloalkyl group; or an aryl group.

According to an exemplary embodiment of the present invention, R1 is a substituted or unsubstituted alkyl group having 1 to 30 carbon atoms; a substituted or unsubstituted cycloalkyl group having 3 to 30 carbon atoms; or a substituted or unsubstituted aryl group having 6 to 30 carbon atoms.

According to an exemplary embodiment of the present invention, R1 is a substituted or unsubstituted alkyl group having 1 to 20 carbon atoms; a substituted or unsubstituted cycloalkyl group having 3 to 20 carbon atoms; or a substituted or unsubstituted aryl group having 6 to 20 carbon atoms.

According to an exemplary embodiment of the present invention, R1 is a substituted or unsubstituted alkyl group having 1 to 10 carbon atoms; a substituted or unsubstituted cycloalkyl group having 3 to 10 carbon atoms; or a substituted or unsubstituted aryl group having 6 to 10 carbon atoms.

According to an exemplary embodiment of the present invention, R1 is a substituted or unsubstituted alkyl group having 1 to 6 carbon atoms; a substituted or unsubstituted monocyclic cycloalkyl group; or a substituted or unsubstituted monocyclic aryl group.

According to an exemplary embodiment of the present invention, R1 is a substituted or unsubstituted alkyl group; a substituted or unsubstituted cycloalkyl group; or an aryl group which is unsubstituted or substituted with one or two or more substituents selected from the group consisting of a halogen group and an alkyl group.

According to an exemplary embodiment of the present invention, R1 is an alkyl group; a cycloalkyl group; or an aryl group which is unsubstituted or substituted with one or two or more substituents selected from the group consisting of a halogen group and an alkyl group.

According to an exemplary embodiment of the present invention, R1 is a substituted or unsubstituted methyl group; a substituted or unsubstituted propyl group; a substituted or unsubstituted butyl group; a substituted or unsubstituted cyclohexyl group; or a phenyl group which is unsubstituted or substituted with one or two or more substituents selected from the group consisting of a halogen group and an alkyl group.

According to an exemplary embodiment of the present invention, R1 is a substituted or unsubstituted methyl group; a substituted or unsubstituted propyl group; a substituted or unsubstituted butyl group; a substituted or unsubstituted cyclohexyl group; or a phenyl group which is unsubstituted or substituted with one or two or more substituents selected from the group consisting of a chloro group, a fluoro group and a methyl group.

According to an exemplary embodiment of the present invention, R1 is a substituted or unsubstituted methyl group; a substituted or unsubstituted isopropyl group; a substituted or unsubstituted tert-butyl group; a substituted or unsubstituted cyclohexyl group; or a phenyl group which is unsubstituted or substituted with one or two or more substituents selected from the group consisting of a chloro group, a fluoro group and a methyl group.

According to an exemplary embodiment of the present invention, R1 is a methyl group; an isopropyl group; a tert-butyl group; a cyclohexyl group; or a phenyl group which is unsubstituted or substituted with one or two or more substituents selected from the group consisting of a chloro group, a fluoro group and a methyl group.

According to an exemplary embodiment of the present invention, R1 is a methyl group; an isopropyl group; a tert-butyl group; a cyclohexyl group; or a phenyl group which is unsubstituted or substituted with one substituent selected from the group consisting of a chloro group, a fluoro group and a methyl group.

According to an exemplary embodiment of the present invention, R2 is -C(=O)Rb; a substituted or unsubstituted alkyl group; a substituted or unsubstituted cycloalkyl group; a substituted or unsubstituted aryl group; or a substituted or unsubstituted heterocyclic group, and Rb is hydrogen; deuterium; a hydroxyl group; a nitrile group; a substituted or unsubstituted alkyl group; a substituted or unsubstituted aryl group; or a substituted or unsubstituted heterocyclic group.

According to an exemplary embodiment of the present invention, R2 is a substituted or unsubstituted alkyl group; a substituted or unsubstituted cycloalkyl group; or a substituted or unsubstituted aryl group.

According to an exemplary embodiment of the present invention, R2 is a substituted or unsubstituted alkyl group having 1 to 30 carbon atoms; a substituted or unsubstituted cycloalkyl group having 3 to 30 carbon atoms; or a substituted or unsubstituted aryl group having 6 to 30 carbon atoms.

According to an exemplary embodiment of the present invention, R2 is a substituted or unsubstituted alkyl group having 1 to 20 carbon atoms; a substituted or unsubstituted cycloalkyl group having 3 to 20 carbon atoms; or a substituted or unsubstituted aryl group having 6 to 20 carbon atoms.

According to an exemplary embodiment of the present invention, R2 is a substituted or unsubstituted alkyl group having 1 to 10 carbon atoms; a substituted or unsubstituted cycloalkyl group having 3 to 10 carbon atoms; or a substituted or unsubstituted aryl group having 6 to 10 carbon atoms.

According to an exemplary embodiment of the present invention, R2 is a substituted or unsubstituted alkyl group; a substituted or unsubstituted cycloalkyl group; or a substituted or unsubstituted monocyclic aryl group.

According to an exemplary embodiment of the present invention, R2 is an alkyl group; a cycloalkyl group; or an aryl group which is unsubstituted or substituted with one or two or more substituents selected from the group consisting of a halogen group, an ether group and an alkyl group.

According to an exemplary embodiment of the present invention, R2 is an alkyl group; a cycloalkyl group; or an aryl group which is unsubstituted or substituted with one or two or more substituents selected from the group consisting of a halogen group, an ether group and an alkyl group having 1 to 10 carbon atoms.

According to an exemplary embodiment of the present invention, R2 is an alkyl group; a cycloalkyl group; or an aryl group which is unsubstituted or substituted with one or two or more substituents selected from the group consisting of a halogen group, an ether group and an alkyl group having 1 to 6 carbon atoms.

According to an exemplary embodiment of the present invention, R2 is an alkyl group; a cycloalkyl group; or an aryl group which is unsubstituted or substituted with one or two or more substituents selected from the group consisting of a halogen group, an ether group and an alkyl group having 1 to 6 carbon atoms.

According to an exemplary embodiment of the present invention, R2 is an alkyl group; a cycloalkyl group; or an aryl group which is unsubstituted or substituted with one or two or more substituents selected from the group consisting of a chloro group, a fluoro group, $-OCH_3$ and a methyl group.

According to an exemplary embodiment of the present invention, R2 is a substituted or unsubstituted methyl group; a substituted or unsubstituted tert-butyl group; a substituted or unsubstituted cyclohexyl group; or a phenyl group which is unsubstituted or substituted with one or two or more substituents selected from the group consisting of a chloro group, a fluoro group, $-OCH_3$ and a methyl group.

According to an exemplary embodiment of the present invention, R2 is a methyl group; a tert-butyl group; a

cyclohexyl group; a phenyl group substituted with a methyl group; a phenyl group substituted with a dimethyl group; a phenyl group substituted with a chloro group; a phenyl group substituted with a fluoro group; a phenyl group substituted with $-OCH_3$; or a phenyl group.

According to an exemplary embodiment of the present invention, R3 and R4 are the same as or different from each other, and are each independently hydrogen; deuterium; a substituted or unsubstituted alkyl group; a substituted or unsubstituted cycloalkyl group; or a substituted or unsubstituted aryl group.

According to an exemplary embodiment of the present invention, R3 and R4 are the same as or different from each other, and are each independently a substituted or unsubstituted cycloalkyl group; or a substituted or unsubstituted aryl group.

According to an exemplary embodiment of the present invention, R3 and R4 are the same as or different from each other, and are each independently a cycloalkyl group; or a substituted or unsubstituted aryl group.

According to an exemplary embodiment of the present invention, R3 and R4 are the same as or different from each other, and are each independently a cycloalkyl group; or an aryl group.

According to an exemplary embodiment of the present invention, R3 and R4 are the same as or different from each other, and are each independently a substituted or unsubstituted cycloalkyl group having 3 to 30 carbon atoms; or a substituted or unsubstituted aryl group having 6 to 30 carbon atoms.

According to an exemplary embodiment of the present invention, R3 and R4 are the same as or different from each other, and are each independently a substituted or unsubstituted cycloalkyl group having 3 to 20 carbon atoms; or a substituted or unsubstituted aryl group having 6 to 20 carbon atoms.

According to an exemplary embodiment of the present invention, R3 and R4 are the same as or different from each other, and are each independently a substituted or unsubstituted cycloalkyl group having 3 to 10 carbon atoms; or a substituted or unsubstituted aryl group having 6 to 10 carbon atoms.

According to an exemplary embodiment of the present invention, R3 and R4 are the same as or different from each other, and are each independently a substituted or unsubstituted cycloalkyl group having 3 to 6 carbon atoms; or a substituted or unsubstituted monocyclic aryl group.

According to an exemplary embodiment of the present invention, R3 and R4 are the same as or different from each other, and are each independently a substituted or unsubstituted cycloalkyl group having 3 to 6 carbon atoms; or a substituted or unsubstituted phenyl group.

According to an exemplary embodiment of the present invention, R3 and R4 are the same as or different from each other, and are each independently a substituted or unsubstituted cyclopropyl group; a substituted or unsubstituted cyclobutyl group; a substituted or unsubstituted cyclopentyl group; a substituted or unsubstituted cyclohexyl group; or a substituted or unsubstituted phenyl group.

According to an exemplary embodiment of the present invention, R3 and R4 are the same as or different from each other, and are each independently a cyclopropyl group; a cyclobutyl group; a cyclopentyl group; a cyclohexyl group; or a substituted or unsubstituted phenyl group.

According to an exemplary embodiment of the present invention, R3 and R4 are the same as or different from each other, and are each independently a cyclopropyl group; a cyclobutyl group; a cyclopentyl group; a cyclohexyl group; or a phenyl group which is unsubstituted or substituted with one or two or more substituents selected from the group consisting of an alkyl group, a halogen group and - ORe'.

According to an exemplary embodiment of the present invention, R3 and R4 are the same as or different from each other, and are each independently a cyclopropyl group; a cyclobutyl group; a cyclopentyl group; a cyclohexyl group; or a phenyl group which is unsubstituted or substituted with one or two or more substituents selected from the group consisting of an alkyl group having 1 to 10 carbon atoms, a halogen group and -ORe'.

According to an exemplary embodiment of the present invention, R3 and R4 are the same as or different from each other, and are each independently a cyclopropyl group; a cyclobutyl group; a cyclopentyl group; a cyclohexyl group; or a phenyl group which is unsubstituted or substituted with one or two or more substituents selected from the group consisting of an alkyl group having 1 to 6 carbon atoms, a halogen group and -ORe'.

According to an exemplary embodiment of the present invention, R3 and R4 are the same as or different from each other, and are each independently a cyclopropyl group; a cyclobutyl group; a cyclopentyl group; a cyclohexyl group; or a phenyl group which is unsubstituted or substituted with one or two or more substituents selected from the group consisting of a methyl group, a chloro group, a fluoro group and -ORe'.

According to an exemplary embodiment of the present invention, Re' is hydrogen; deuterium; or a substituted or unsubstituted alkyl group.

According to an exemplary embodiment of the present invention, Re' is a substituted or unsubstituted alkyl group.

According to an exemplary embodiment of the present invention, Re' is a substituted or unsubstituted alkyl group having 1 to 10 carbon atoms.

According to an exemplary embodiment of the present invention, Re' is a substituted or unsubstituted alkyl group having 1 to 6 carbon atoms.

According to an exemplary embodiment of the present invention, Re' is a substituted or unsubstituted methyl group.

According to an exemplary embodiment of the present invention, Re' is a methyl group.

According to an exemplary embodiment of the present invention, R3 and R4 are the same as or different from each other, and are each independently a cyclopropyl group; a cyclobutyl group; a cyclopentyl group; a cyclohexyl group; or a phenyl group which is unsubstituted or substituted with one or two or more substituents selected from the group consisting of a methyl group, a chloro group, a fluoro group and $-OCH_3$.

According to an exemplary embodiment of the present invention, R3 and R4 are the same as or different from each other, and are each independently a cyclopropyl group; a cyclobutyl group; a cyclopentyl group; a cyclohexyl group; a phenyl group substituted with a methyl group; a phenyl group substituted with a dimethyl group; a phenyl group substituted with a chloro group; a methyl group substituted with a fluoro group; a phenyl group substituted with $-OCH_3$; or a phenyl group.

According to an exemplary embodiment of the present invention, R3 and R4 are the same as or different from each other, and are each independently any one selected from the following structures.

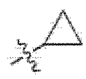 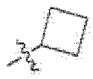 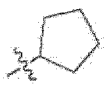 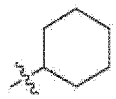 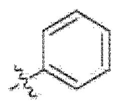

In the structures,

the structures are unsubstituted or substituted with deuterium; a halogen group; -ORe'; or a substituted or unsubstituted alkyl group,

Re' is hydrogen; deuterium; or a substituted or unsubstituted alkyl group, and

" 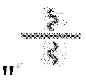 "

means a site linked to Chemical Formula H.

According to an exemplary embodiment of the present invention, R3 and R4 are the same as or different from each other, and are each independently any one selected from the following structures.

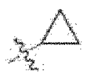 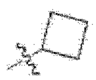 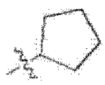 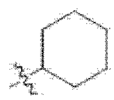 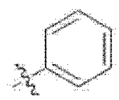

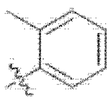 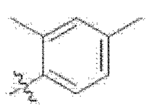 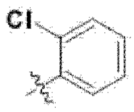 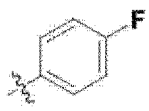 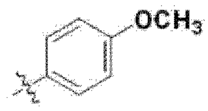

In the structures,

" 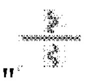 "

means a site linked to Chemical Formula H.

According to an exemplary embodiment of the present invention, R3 and R4 are the same as each other.

According to an exemplary embodiment of the present invention, Chemical Formula H is any one selected from the group consisting of the following compounds.

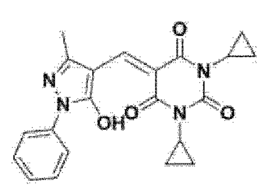
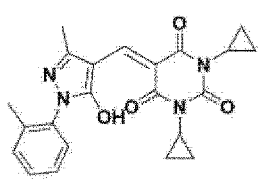
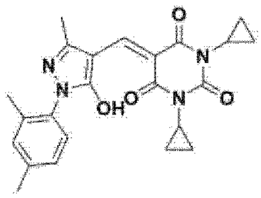
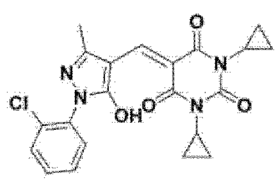

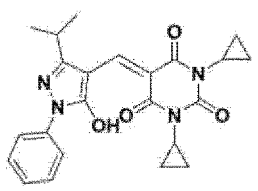
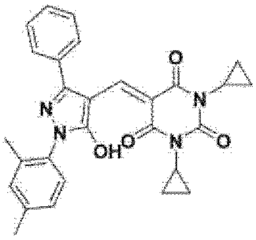
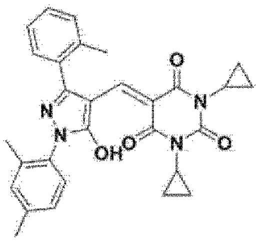
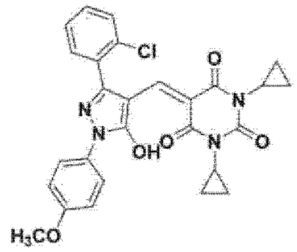

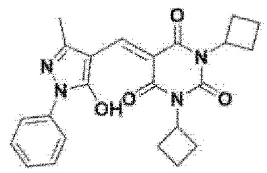
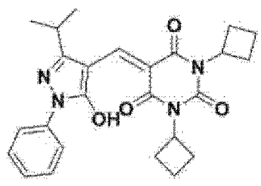
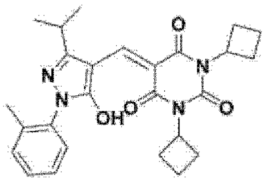
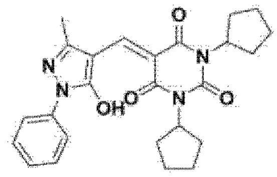

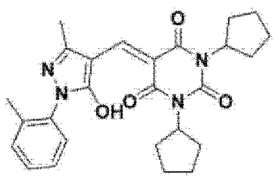
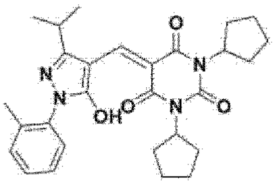
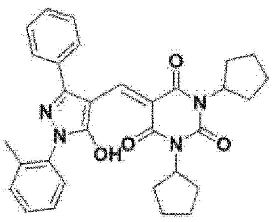
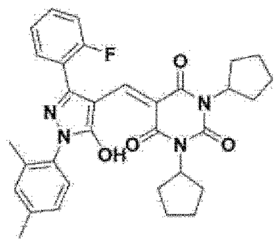

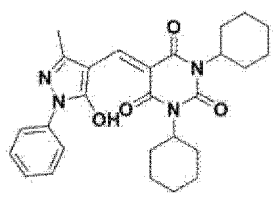
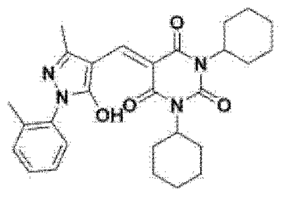
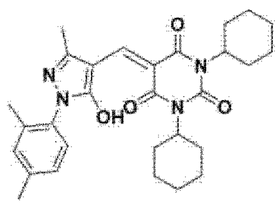
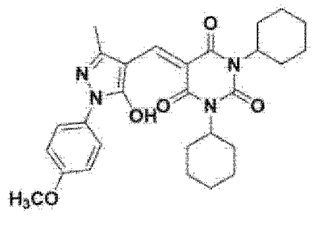

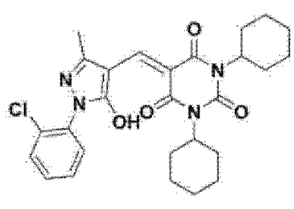
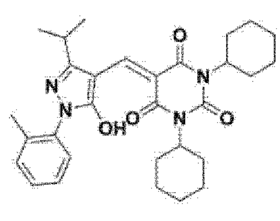
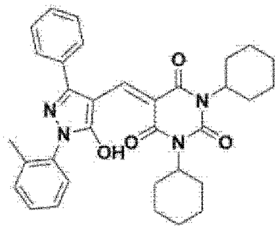
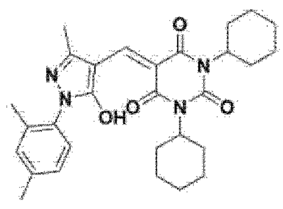

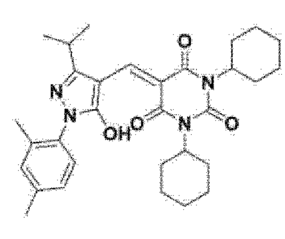
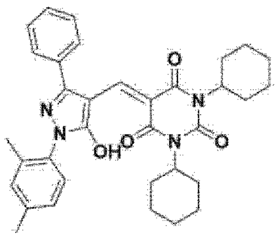
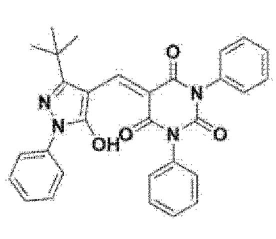
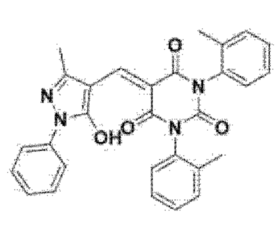
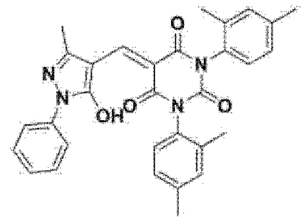
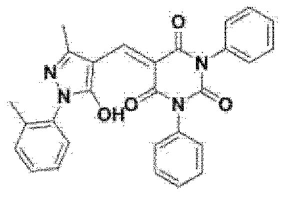
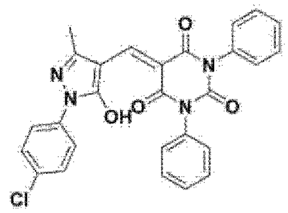
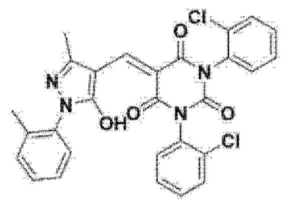
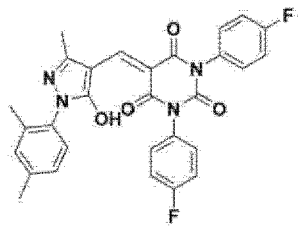
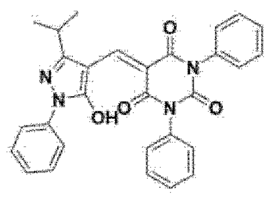
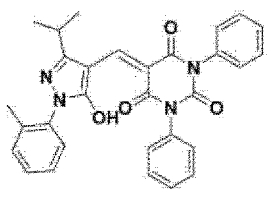
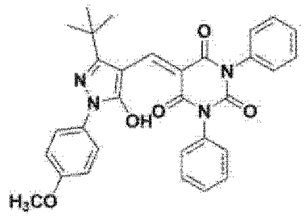
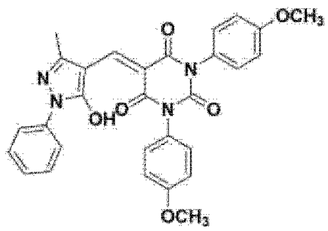
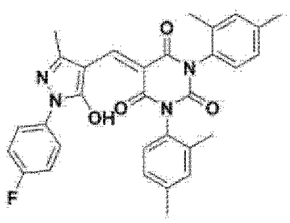
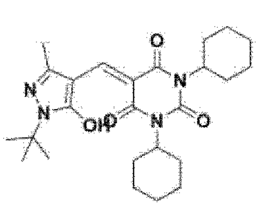
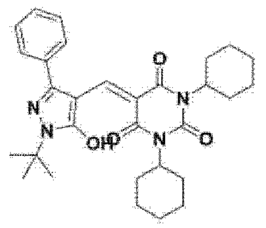
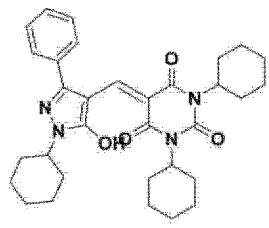
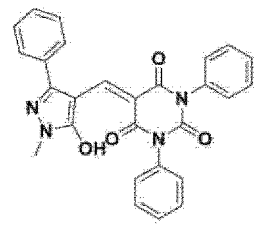
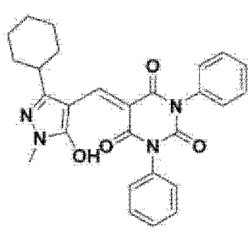
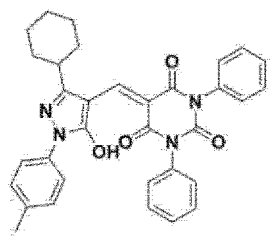
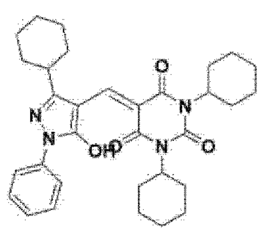

An exemplary embodiment of the present invention provides an adhesive composition including: an adhesive material; and the compound represented by Chemical Formula H.

According to an exemplary embodiment of the present invention, the adhesive composition including the compound represented by Chemical Formula H may be used for an adhesive film, an optical film and/or an electronic device

including the adhesive film or optical film instead of a polarizing plate. Accordingly, the material cost may be reduced or the flexibility of the device structure design may be increased compared to the case where the polarizing plate is used. In addition, when the adhesive composition including the compound of Chemical Formula H is used for an optical film and/or an electronic device including the optical film instead of a polarizing plate, a high power for reinforcing low brightness is not required because it is possible to prevent the brightness from being reduced by a circular polarizing plate when a polarizing plate is used.

Furthermore, in order to adjust the amount of light exposed to an electronic device, the adhesive composition according to an exemplary embodiment of the present invention used instead of a polarizing plate may adjust not only electrode reflection occurring on the electrode part in the device, but also surface reflection occurring on the surface of the device, so that the hue of light emitting color may be flexibly adjusted compared to the polarizing plate.

And, the adhesive composition of the present invention, which includes a pyrazole derivative compound may secure excellent solubility to solvent. Further, since the adhesive composition of the present invention has a low cohesive force, the adhesive composition has an advantage in that the composition can be dispersed as fine particles and there is less color imbalance or particle scattering. Accordingly, the adhesive composition of the present invention, which includes the pyrazole derivative compound may absorb light with a wavelength in a specific range.

According to an exemplary embodiment of the present invention, a publicly-known adhesive material typically used in the art can be used as long as the adhesive material is a material that can be used for a film for a display while imparting adhesiveness. Examples of the adhesive material include an acrylic polymer, an acrylate-based copolymer, a silicone-based polymer, polyester, polyurethane, polyether, an epoxy resin, and the like, but are not limited thereto.

According to an exemplary embodiment of the present invention, the adhesive material may be an acrylate-based resin. The acrylate-based resin may be an acrylate-based polymer or an acrylate-based copolymer. Examples of the acrylate-based copolymer may include butyl acrylate/hydroxyethyl acrylate, but are not limited to the examples.

According to preferred exemplary embodiments of the present invention, as the adhesive material, AD-701 manufactured by LG Chem. may be used, but the adhesive material is not limited thereto.

According to an exemplary embodiment of the present invention, the adhesive material may be included in an amount of 93 parts by weight to 97 parts by weight; or 94 parts by weight to 96 parts by weight based on 100 parts by weight of a solid content except for a solvent in the adhesive composition. According to a preferred exemplary embodiment of the present invention, the adhesive material may be included in an amount of 95 parts by weight to 96 parts by weight based on 100 parts by weight of a solid content except for a solvent in the adhesive composition.

According to an exemplary embodiment of the present invention, the compound of Chemical Formula H may be included in an amount of 0.1 part by weight to 3 parts by weight; 0.1 part by weight to 0.5 part by weight; or 0.2 part by weight to 0.35 part by weight based on 100 parts by weight of a solid content except for a solvent in the adhesive composition. According to a preferred exemplary embodiment of the present invention, the compound of Chemical Formula H may be included in an amount of 0.25 part by weight to 0.35 part by weight based on 100 parts by weight of a solid content except for a solvent in the adhesive composition.

According to another exemplary embodiment of the present invention, the compound of Chemical Formula H may be used with other types of dyes. A dye which may be used with the compound of Chemical Formula H can be used as long as the dye can be typically used in the art. As a specific example of a dye which may be used with the compound of Chemical Formula H, it is possible to select one or more from the group consisting of a metal-complex-based compound; an azo-based compound; a metal azo-based compound; a quinophthalone-based compound; an isoindoline-based compound; a methine-based compound; a phthalocyanine-based compound; a metal phthalocyanine-based compound; a porphyrin-based compound; a metal porphyrin-based compound; a tetra azaporphyrin-based compound; a metal tetra aza porphyrin-based compound; a cyanine-based compound; a xanthene-based compound; a metal dipyrromethene-based compound; a boron dipyrromethene-based compound; a metal dipyrromethene-based compound; an anthraquinone-based compound; a diketopyrrolopyrrole-based compound; a triarylmethane-based compound; and a perylene-based compound. However, the example is not limited thereto.

According to an exemplary embodiment of the present invention, the adhesive composition may further include a solvent. As the solvent, it is possible to apply a compound known to enable the formation of an adhesive composition in the art to which the present invention pertains without particular limitation. For example, the solvent may be one or more compounds selected from the group consisting of esters, ethers, ketones, aromatic hydrocarbons, and sulfoxides.

The ester solvent may be, for example, ethyl acetate, n-butyl acetate, isobutyl acetate, amyl formate, isoamyl acetate, isobutyl acetate, butyl propionate, isopropyl butyrate, ethyl butyrate, butyl butyrate, methyl lactate, ethyl lactate, gamma-butyrolactone, epsilon-caprolactone, delta-valerolactone, alkyl oxyacetate (for example: methyl oxyacetate, ethyl oxyacetate, butyl oxyacetate (for example, methyl methoxyacetate, ethyl methoxyacetate, butyl methoxyacetate, methyl ethoxyacetate, ethyl ethoxyacetate, and the like)), 3-oxypropionic acid alkyl esters (for example: methyl 3-oxypropionate, ethyl 3-oxypropionate, and the like (for example, methyl 3-methoxypropionate, ethyl 3-methoxypropionate, methyl 3-ethoxypropionate, ethyl 3-ethoxypropionate, and the like)), 2-oxypropionic acid alkyl esters (for example: methyl 2-oxypropionate, ethyl 2-oxypropionate, propyl 2-oxypropionate, and the like (for example, methyl 2-methoxypropionate,

ethyl 2-methoxypropionate, propyl 2-methoxypropionate, methyl 2-ethoxypropionate, ethyl 2-ethoxypropionate)), methyl 2-oxy-2-methylpropionate and ethyl 2-oxy-2-methylpropionate (for example, methyl 2-methoxy-2-methylpropionate, ethyl 2-ethoxy-2-methylpropionate, and the like), methyl pyruvate, ethyl pyruvate, propyl pyruvate, methyl acetoacetate, ethyl acetoacetate, methyl 2-oxobutyrate, ethyl 2-oxobutyrate, or the like.

The ether solvent may be, for example, diethylene glycol dimethyl ether, tetrahydrofuran, ethylene glycol monomethyl ether, ethylene glycol monoethyl ether, methyl cellosolve acetate, ethyl cellosolve acetate, diethylene glycol monomethyl ether, diethylene glycol monoethyl ether, diethylene glycol monobutyl ether, propylene glycol monomethyl ether, propylene glycol monomethyl ether acetate, propylene glycol monoethyl ether acetate, propylene glycol monopropyl ether acetate, or the like.

The ketone solvent may be, for example, methyl ethyl ketone, cyclohexanone, cyclopentanone, 2-heptanone, 3-heptanone, N-methyl-2-pyrrolidone, or the like.

The aromatic hydrocarbon solvent may be, for example, toluene, xylene, anisole, limonene, or the like. The sulfoxide solvent may be, for example, dimethyl sulfoxide or the like.

Although the solvent is exemplified as described above, any solvent capable of dissolving or dispersing the compound of Chemical Formula H of the present invention is sufficient, and the solvent is not limited to the above-exemplified solvent.

According to a preferred exemplary embodiment of the present invention, the solvent included in the adhesive composition is ethyl acetate or methyl ethyl ketone.

Further, the solvents may be used either alone or in a mixture of two or more solvents. For example, according to an exemplary embodiment of the present invention, the solvent included in the adhesive composition is a mixed solution of ethyl acetate or methyl ethyl ketone. In this case, the solvent mixed solution may be used by mixing at a ratio of 1: 3 to 3: 1, but this is merely an example, and the mixing ratio is not limited to the above example.

According to a preferred exemplary embodiment of the present invention, as the solvent, methyl ethyl ketone (MEK) may be used, but the solvent is not limited thereto. As a preferred example, the solubility of the compound represented by Chemical Formula H in methyl ethyl ketone (MEK) is more than 0.1 wt.%. As a further preferred example, the solubility of the compound represented by Chemical Formula H in methyl ethyl ketone (MEK) is 0.3 wt.% or more.

According to an exemplary embodiment of the present invention, the solvent may be included in an amount of 10 parts by weight to 70 parts by weight; 10 parts by weight to 50 parts by weight; or 20 parts by weight to 40 parts by weight based on the total parts by weight of the adhesive composition. According to a preferred exemplary embodiment of the present invention, the solvent may be included in an amount of 20 parts by weight to 30 parts by weight based on the total parts by weight of the adhesive composition, but the content is not limited thereto.

According to an exemplary embodiment of the present invention, the adhesive composition may further include other additives. Examples of the other additives include a cross-linking agent, a silane-based coupling agent, a catalyst, an antioxidant, an antistatic agent, a light stabilizer, and the like, but are not limited thereto.

According to an exemplary embodiment of the present invention, the cross-linking agent may induce a cross-linking reaction between an alkali-soluble polyimide resin and other additive components to increase the heat resistance and chemical resistance of a produced film. In this case, as the cross-linking agent, a compound including a functional group such as an acrylic group and an isocyanate group may be used. In addition, examples of the cross-linking agent include a thermal cross-linking agent, and as the thermal cross-linking agent, a compound including a heat-reactive functional group such as a methylol group and an epoxy group may be used. As a specific example, it is possible to use DML-PC, DML-PEP, DML-OC, DML-OEP, DML-34X, DML-PTBP, DML-PCHP, DML-OCHP, DML-PFP, DML-PSBP, DML-POP, DML-MBOC, DML-MBPC,DML-MTrisPC, DML-BisOC-Z, DML-BisOCHP-Z, DML-BPC, DML-BisOC-P, DMOM-PC, DMOM-PTBP, DMOM-MBPC, TriML-P,TriML-35XL, TML-HQ, TML-BP, TML-pp-BPF, TML-BPE, TML-BPA, TML-BPAF, TML-BPAP, TMOM-BP, TMOM-BPE, TMOM-BPA, TMOM-BPAF, TMOM-BPAP, HML-TPPHBA, HML-TPHAP, HMOM-TPPHBA, HMOM-TPHAP (all are trade names, manufactured by Honshu Chemical Industry Co., Ltd.), "NIKALAC" (registered trademark) MX-290, "NIKALAC" (registered trademark) MX-280, "NIKALAC" (registered trademark) MX-270,"NIKALAC" (registered trademark) MX-279, "NIKALAC" (registered trademark) MW-100LM, "NIKALAC" (registered trademark) MX-750LM (all are trade names, manufactured by Sanwa Chemical Co., Ltd.), T39M (Soken Chemical & Engineering Co., Ltd.), and the like, which are cross-linking agents generally used in the art.

The cross-linking agent may be included in an amount of 0.05 part by weight to 0.2 part by weight based on 100 parts by weight of a solid content of the adhesive composition. As a preferred example, the cross-linking agent may be included in an amount of 0.07 part by weight to 0.15 part by weight based on 100 parts by weight of the adhesive composition, but the content is not limited thereto.

The silane-based coupling agent may be used, for example, to improve the dispersibility of a thermally conductive filler such as alumina, and various types of silane-based coupling agents known in the art can be used without limitation as long as the silane-based coupling agent can exhibit the action as described above. The silane-based coupling agent means a compound including a hydrolyzable silyl group or silanol group. Further, the silane-based coupling agent may increase heat resistance and chemical resistance by increasing the mutual adhesion between a film produced by curing

and one specific surface of the substrate. As an example of the silane-based coupling agent, it is possible to select and use one or more from octyltrimethoxysilane, dodecyltrimethoxysilane, octadecytrimethoxysilane, and the like, but the silane-based coupling agent is not limited thereto.

According to preferred exemplary embodiments of the present invention, as the silane-based coupling agent, T-789J (Soken Chemical & Engineering Co., Ltd.) may be used, but the silane-based coupling agent is not limited thereto.

The content of the silane-based coupling agent may be 0.1 part by weight to 0.4 part by weight; or 0.1 part by weight to 0.3 part by weight based on 100 parts by weight of a solid content of the adhesive composition. As a preferred example, the content of the silane-based coupling agent may be 0.15 part by weight to 0.3 part by weight based on 100 parts by weight of a solid content of the adhesive composition, but is not limited thereto.

The antioxidant may serve to prevent a chain reaction in which radicals are generated during the formation of a polymer film. In this case, the antioxidant may include a phenolic antioxidant, and the like, and 2,2-thiobis(4-methyl-6-t-butylphenol), or 2,6-g,t-butylphenol, and the like, which are antioxidants generally used in the art, may be used, and as the UV absorbent, 2-(3-t-butyl-5-methyl-2-hydroxyphenyl)-5-chloro-benzotriazole, or alkoxy benzophenone, and the like may be used, but the UV absorbent is not limited thereto. According to preferred exemplary embodiments of the present invention, as the antioxidant, Kinox-80 (Hannong Chemicals Inc.) may be used, but the antioxidant is not limited thereto.

The content of the antioxidant may be 0.2 part by weight to 0.8 part by weight; or 0.3 part by weight to 0.7 part by weight based on 100 parts by weight of a solid content of the adhesive composition. As a preferred example, the antioxidant may be included in an amount of 0.4 part by weight to 0.6 part by weight based on 100 parts by weight of a solid content of the adhesive composition, but the content is not limited thereto.

The antistatic agent is included in the adhesive composition, and thus serves to impart an antistatic performance, and all publicly-known antistatic agents may be used. As the antistatic agent, for example, an ionic compound may be used. As the ionic compound, for example, a metal salt or an organic salt may be used. The metal salt ionic compound may include, for example, an alkali metal cation or an alkaline earth metal cation. As the cation, one or two or more such as a lithium ion ($Li^+$), a sodium ion ($Na^+$), a potassium ion ($K^+$), a rubidium ion ($Rb^+$), a cesium ion ($Cs^+$), a beryllium ion ($Be^{2+}$), a magnesium ion ($Mg^{2+}$), a calcium ion ($Ca^{2+}$), a strontium ion ($Sr^{2+}$) and a barium ion ($Ba^{2+}$) may be exemplified, and for example, it is possible to use one or two or more of a lithium ion, a sodium ion, a potassium ion, a magnesium ion, a calcium ion and a barium ion, or a lithium ion in consideration of ion stability and mobility. As the anion included in the metal salt, $PF_6^-$, $AsF^-$, $NO_2^-$, fluoride (F-), chloride (Cl-), bromide ($Br^-$), iodide ($I^-$), perchlorate ($ClO_4^-$), hydroxide ($OH^-$), carbonate ($CO_3^{2-}$), nitrate ($NO_3^-$), trifluoromethanesulfonate ($CF_3SO_3^-$), sulfonate ($SO_4^-$), hexafluorophosphate ($PF_6^-$), methylbenzenesulfonate ($CH_3(C_6H_4)SO_3^-$), p-toluenesulfonate ($CH_3C_6H_4SO_3^-$), tetraborate ($B_4O_7^{2-}$), carboxybenzenesulfonate (COOH ($C_6H_4$) $SO_3^-$), trifluoromethanesulfonate ($CF_3SO_2^-$), benzoate ($C_6H_5COO^-$), acetate ($CH_3COO^-$), trifluoroacetate ($CF_3COO^-$), tetrafluoroborate ($BF_4^-$), tetrabenzylborate ($B(C_6H_5)_4^-$), trispentafluoroethyl trifluorophosphate ($P(C_2F_5)_3F_3^-$), or the like may be exemplified. According to a preferred exemplary embodiment of the present invention, as the antistatic agent, FC-4400 (3M) may be used, but the antistatic agent is not limited thereto.

The content of the antistatic agent may be 0.5 part by weight to 2.5 parts by weight; or 0.7 part by weight to 2.3 parts by weight based on 100 parts by weight of a solid content of the adhesive composition. As a preferred example, the content of the antistatic agent may be 1 part by weight to 2 parts by weight based on 100 parts by weight of a solid content of the adhesive composition, but is not limited thereto.

The light stabilizer is a material which is not aggregated even when an adhesive agent is left to stand under high temperature conditions, and thus does not induce a phenomenon in which the concentration of an antistatic agent to be described below in the aggregated cluster increases, and can significantly improve the storage stability of the adhesive composition by preventing a problem in that a bonding site is degraded by heat to generate radicals, and a publicly-known light stabilizer may be used. According to a preferred exemplary embodiment of the present invention, as the light stabilizer, a hindered amine compound may be used, and specifically, Tinuvin 123 (BASF SE) may be used, but the light stabilizer is not limited thereto.

The content of the light stabilizer may be 1 part by weight to 4 parts by weight; or 1 part by weight to 3 parts by weight based on 100 parts by weight of a solid content of the adhesive composition. As a preferred example, the content of the light stabilizer may be 1.5 parts by weight to 2.5 parts by weight based on 100 parts by weight of a solid content of the adhesive composition, but is not limited thereto.

As the catalyst, a material that adjusts the rate of a reaction for preparing an adhesive composition according to an exemplary embodiment of the present invention is used. According to an exemplary embodiment of the present invention, as the catalyst, dibutyltin dilaurate (Sigma-Aldrich) may be used, but the catalyst is not limited thereto.

The content of the catalyst may be 0.005 part by weight to 0.02 part by weight; or 0.006 part by weight to 0.019 part by weight based on 100 parts by weight of a solid content of the adhesive composition. As a preferred example, the content of the catalyst may be 0.007 part by weight to 0.015 part by weight based on 100 parts by weight of a solid content of the adhesive composition, but is not limited thereto.

An exemplary embodiment of the present invention provides an adhesive composition which further comprises

a cross-linking agent, a silane-based coupling agent, an antioxidant, an antistatic agent, a light stabilizer and a catalyst, in which based on 100 parts by weight of a solid content of the adhesive composition, a content of the cross-linking agent is 0.05 part by weight to 0.2 part by weight, a content of the silane-based coupling agent is 0.1 part by weight to 0.4 part by weight, a content of the antioxidant is 0.2 part by weight to 0.8 part by weight, a content of the antistatic agent is 0.5 part by weight to 2.5 parts by weight, a content of the light stabilizer is 1 part by weight to 4 parts by weight, and a content of the catalyst is 0.005 part by weight to 0.02 part by weight.

An exemplary embodiment of the present invention provides an adhesive film including the above-described adhesive composition.

According to an exemplary embodiment of the present invention, the adhesive film may be used as an optical film used for a display, an electronic device, and the like. Examples of the optical film include a phase difference film, an anti-reflection film, an anti-glare film, a UV-light absorbing film, an infrared light absorbing film, an optical compensation film, a brightness enhancement film, and the like.

According to an exemplary embodiment of the present invention, the adhesive film including the adhesive composition may include the adhesive composition as it is.

According to another exemplary embodiment of the present invention, the adhesive film including the adhesive composition may include the adhesive composition in a state where the solvent has been removed by drying.

According to still another exemplary embodiment of the present invention, the adhesive film including the adhesive composition may include a cured product of the adhesive composition.

According to an exemplary embodiment of the present invention, under conditions of light fastness Are (lx) * Tre (time), a change in transmittance of the adhesive film including the above-described adhesive composition for light with a wavelength of 390 to 450 nm is 3% or less.

According to an exemplary embodiment of the present invention, Are may be 50,000 to 60,000 lx; or 52,000 to 58,000 lx.

According to an exemplary embodiment of the present invention, Tre may be 36 to 60 hours; or 40 to 52 hours.

According to an exemplary embodiment of the present invention, under conditions of light fastness 55,600 lx * 48 hours, a change in transmittance of the adhesive film including the above-described adhesive composition for light with a wavelength of 390 to 450 nm is 3% or less. According to a preferred exemplary embodiment of the present invention, under conditions of light fastness 55,600 lx * 48 hours, a change in transmittance of the adhesive film including the above-described adhesive composition for light with a wavelength of 390 to 450 nm is 1.5% or less.

In the present invention, transmittance means transmittance with respect to light.

According to an exemplary embodiment of the present invention, under conditions of heat resistance 50 to 100°C and for 500 hours, a change in transmittance of the adhesive film including the above-described adhesive composition for light with a wavelength of 390 to 450 nm is 3% or less. According to another exemplary embodiment of the present invention, under conditions of heat resistance 80°C and for 500 hours, a change in transmittance of the adhesive film including the above-described adhesive composition for light with a wavelength of 390 to 450 nm is 3% or less. According to a preferred exemplary embodiment of the present invention, under conditions of heat resistance 80°C and for 500 hours, a change in transmittance of the adhesive film including the above-described adhesive composition for light with a wavelength of 390 to 450 nm is 1.5% or less.

According to an exemplary embodiment of the present invention, under conditions of moisture resistance 60°C, 80 to 99% RH (relative humidity) and for 500 hours, a change in transmittance of the adhesive film including the above-described adhesive composition for light with a wavelength of 390 to 450 nm is 3% or less. According to another exemplary embodiment of the present invention, under conditions of moisture resistance 60°C, 90% RH and for 500 hours, a change in transmittance of the adhesive film including the above-described adhesive composition for light with a wavelength of 390 to 450 nm is 3% or less. According to a preferred exemplary embodiment of the present invention, under the same moisture resistance conditions, a change in transmittance of the adhesive film including the above-described adhesive composition for light with a wavelength of 390 to 450 nm is 1.5% or less.

According to an exemplary embodiment of the present invention, under conditions of light fastness 55,600 lx * 48 hours; under conditions of heat resistance 80°C and 500 hours; or under conditions of moisture resistance 60°C, 90% RH and 500 hours, a change in transmittance of the adhesive film including the above-described adhesive composition for light with a wavelength of 390 to 450 nm is 3% or less. According to a preferred exemplary embodiment of the present invention, under conditions of light fastness 55,600 lx * 48 hours; under conditions of heat resistance 80°C and 500 hours; or under conditions of moisture resistance 60°C, 90% RH and 500 hours, a change in transmittance of the adhesive film including the above-described adhesive composition for light with a wavelength of 390 to 450 nm is 1.5% or less.

The change in transmittance for light may be derived from the transmittance values before and after the adhesive film including the above-described adhesive composition is exposed to light in a specific wavelength range under specific conditions As a specific example, the value of a change in transmittance of the adhesive film including the above-described adhesive composition for light with a wavelength of 390 nm under conditions of light fastness 55,600 lx * 48

hours may be derived from the resulting value obtained by dividing the absolute value of the difference between transmittance values of the adhesive film including the above-described adhesive composition measured before and after conditions of light fastness 55,600 lx * 48 hours by the transmittance value measured after the conditions, for light with a wavelength of 390 nm. The transmittance for light may be measured by a UV-vis apparatus. As a specific apparatus, a UV-vis apparatus (Shimazu UV-3600) may be used, but is not limited thereto.

Since the compound according to an exemplary embodiment of the present invention may improve the light blocking efficiency or reduce the reflectance to external light by strongly absorbing light, the compound is excellent in wavelength compatibility in being included in an optical film. The wavelength compatibility can be confirmed by the wavelength distribution and the optimization appearing in the UV-Vis spectrum. For example, FIG. 2 illustrates the solution-phase UV-Vis spectra of Compound P3 according to an exemplary embodiment of the present invention and Comparative Compound Z2 used in Comparative Example 2. In FIG. 2, the dotted line is the solution-phase UV-Vis spectrum for Comparative Compound Z2, and the solid line is the solution-phase UV-Vis spectrum for Compound P3 according to an exemplary embodiment of the present invention. It can be confirmed that Compound P3 of the present invention has a narrower wavelength distribution than Comparative Compound Z2 used in Comparative Example 2 and is optimized, and thus has excellent wavelength compatibility.

According to an exemplary embodiment of the present invention, the adhesive film is used for a display, an electronic device, and the like instead of a polarizing plate to more effectively decrease a change in transmittance for each wavelength than an optical film including a polarizing plate. Further, the adhesive film includes a composition including the compound of Chemical Formula H, and thus more effectively decreases a change in transmittance at each wavelength than an optical film including another composition. Accordingly, the hue of light emitting color may be flexibly adjusted.

According to an exemplary embodiment of the present invention, the adhesive film including the adhesive composition may have a form in which the adhesive composition is laminated and/or applied onto one surface of a base film.

According to an exemplary embodiment of the present invention, the base film may be selected from the group consisting of polyethylene terephthalate (PET), polyester, polycarbonate (PC), polyimide (PI), polyethylene naphthalate (PEN), polyether ether ketone (PEEK), polyarylate (PAR), polycycloolefin (PCO), polynorbornene, polyethersulphone (PES), and a cycloolefin polymer (COP).

Further, it is preferred that the base film is transparent. The fact that the base film is transparent described here indicates that the light transmittance of visible light in a wavelength range of 400 to 700 nm is 80% or more. When the base film has the above range, the laminated adhesive film has a characteristic that the adhesive film can be thinned.

According to an exemplary embodiment of the present invention, the adhesive film including the adhesive composition has a thickness of 10 to 40 um; 15 to 30 um; 20 to 25 um. According to a preferred exemplary embodiment of the present invention, the adhesive film including the adhesive composition has a thickness of 22 to 23 um.

According to an exemplary embodiment of the present invention, the adhesive film may be prepared by applying the adhesive composition onto a base film using a bar coater.

According to an exemplary embodiment of the present invention, the adhesive film including the adhesive composition may further include other films. Examples of the other films include a release film, an anti-reflection film, a binder film, and the like.

As a specific example, FIG. 1 illustrates a film in which a glass (1), an adhesive film (2), and a binder resin film (3) are sequentially laminated according to an exemplary embodiment of the present invention.

According to an exemplary embodiment of the present invention, the release film may be included on the other surface other than one surface of the base film on which the adhesive composition is laminated and/or applied.

As the release film, a hydrophobic film may be used, the release film is a layer for protecting an adhesive sheet having a very small thickness and refers to a transparent layer which is attached to one surface of an adhesive sheet, and it is possible to use a film which is excellent in mechanical strength, heat stability, moisture shielding property, isotropy, and the like. For example, it is possible to use an acetate-based resin film such as triacetyl cellulose (TAC), a polyester-based resin film, a polyether sulfone-based resin film, a polycarbonate-based resin film, a polyamide-based resin film, a polyimide-based resin film, a polyolefin-based resin film, a cycloolefin-based resin film, a polyurethane-based resin film, an acrylic resin film, and the like, but the release film can be used as long as the release film is a commercially available silicone-treated release film, and is not limited to the above example.

According to an exemplary embodiment of the present invention, the anti-reflection film serves to improve the reflection color tone by adjusting the degree of light reflection generated from an external light source.

According to an exemplary embodiment of the present invention, the anti-reflection film has a minimum reflection wavelength of 550 nm or less; or 530 nm or less. According to a preferred exemplary embodiment of the present invention, the anti-reflection film has a minimum reflection wavelength of 500 nm or less.

A material for the anti-reflection film can be appropriately selected in order to satisfy the physical properties of the minimum reflection wavelength. For example, the anti-reflection film may include a low refractive layer. For example,

the minimum reflection wavelength of the anti-reflection film tends to move to a longer wavelength as the thickness of the low refractive layer is increased, and to a shorter wavelength as the thickness of the low refractive layer is decreased. For example, the minimum reflectance of the anti-reflection film tends to decrease as the refractive index of a low refractive material decreases.

The low refractive layer may include a low refractive material. In one example, the low refractive material may be low refractive inorganic particles. In another example, low refractive inorganic particles may be silica-based particles. Examples of the silica-based particles include hollow silica, mesoporous silica, and the like, but are not limited thereto. In still another example, as the low refractive inorganic particles, magnesium fluoride ($MgF_2$) may be used.

According to an exemplary embodiment of the present invention, the low refractive layer may further include a binder resin film. The low refractive inorganic particles may be present in a dispersed state in a binder resin film.

According to an exemplary embodiment of the present invention, examples of the binder resin film include a cellulose triacetate (TAC) film, and the like, but are not limited thereto, and a publicly-known binder resin film typically used in the art may be used.

According to an exemplary embodiment of the present invention, provided is an electronic device including the above-described adhesive composition or the above-described adhesive film.

The electronic device may include all of the interlayer insulation film of a semiconductor device, a color filter, a black matrix, an overcoat, a column spacer, a passivation film, a buffer coating film, a multilayer printed board insulation film, a cover coat of a flexible copper-clad board, a buffer coating film, a multilayer printed board insulation film, a solder resist film, an insulation film of OLED, a protection film of a thin-film transistor of a liquid crystal display device, an electrode protection film and a semiconductor protection film of an organic EL device, an OLED insulation film, an LCD insulation film, a semiconductor insulation film, a display device, and the like, but is not limited thereto.

According to an exemplary embodiment of the present invention, provided is an organic light emitting display including: an adhesive film including the above-described adhesive composition; a substrate provided on one surface of the adhesive film; and an organic light emitting layer provided on a surface opposite to a surface of the substrate brought into contact with the adhesive film.

[Mode for Invention]

Hereinafter, the present invention will be described in detail with reference to Examples for specifically describing the present invention. However, the Examples according to the present invention may be modified into various different forms, and it should not be interpreted that the scope of the present invention is limited to the Examples to be described below. The Examples of the present invention are provided for more completely explaining the present invention to the person with ordinary skill in the art.

<Synthesis Examples>

(1) Synthesis of Intermediate A

[Reaction Scheme 1]

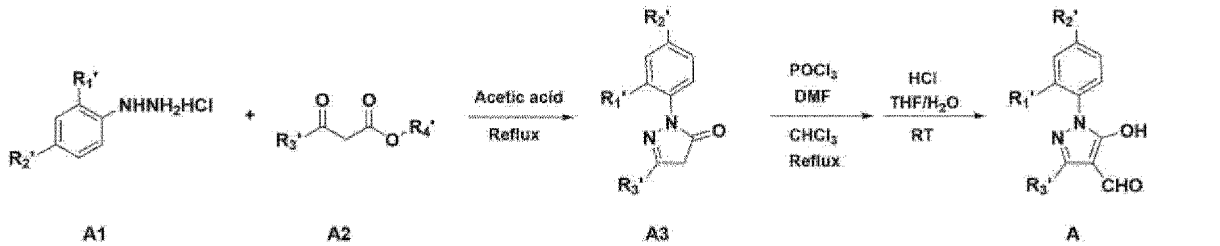


A1 A2 A3 A

A1: $R_1'$ = H, $CH_3$, Cl; $R_2'$ = H, $CH_3$

A2: $R_3'$ = $CH_3$, $(CH_3)_2CH$, $C_6H_5$; $R_4'$ = $CH_3$, $CH_3CH_2$

A: $R_1'$ = H, $CH_3$, Cl; $R_2'$ = H, $CH_3$; $R_3'$ = $CH_3$, $(CH_3)_2CH$, $C_6H_5$

1) Synthesis of Monomer A3

After 1 eq of Monomer A1 and 1.1 eq of Monomer A2 were diluted in 12 eq of an acetic acid solvent, the resulting

mixture was heated and stirred under nitrogen. After the reaction was completed, extraction was performed by adding a water and chloroform solvent thereto. During the extraction, the pH of the solution was adjusted to 6 using potassium acetate. After water was removed by adding anhydrous magnesium sulfate to the extracted organic solvent, the organic solvent was condensed, a precipitate was formed in the condensed organic layer using chloroform and hexane, and the next reaction was performed by securing the precipitated solid through a vacuum filter.

2) Synthesis of Intermediate A

After stirring was performed for 30 minutes by putting 2 eq of dimethylformamide into the chloroform solvent while maintaining the temperature at 0°C under nitrogen, 1.2 eq of $POCl_3$ was slowly added dropwise thereto. After the addition of $POCl_3$ was completed, the temperature was maintained at low temperature for 30 minutes, and then the mixture was stirred at room temperature for 30 minutes by adding 1 eq of Monomer A3 thereto, and then heated. After the reaction was completed, extraction was performed using an aqueous solution of chloroform and KOAc, moisture was removed by adding anhydrous magnesium sulfate to the extracted organic solvent layer, and the solvent was condensed. The condensed solution was diluted in a THF solvent, and an additional reaction was performed by adding a 1 M HCl solution thereto. After the completion of the reaction was confirmed by TLC, extraction was performed with chloroform and water, and moisture was removed by adding anhydrous magnesium sulfate to the extracted chloroform layer. Intermediate A was secured by condensing the organic layer from which moisture was removed. The following Intermediates A4 to A13 were secured by changing the type of substituent to be introduced into Intermediate A.

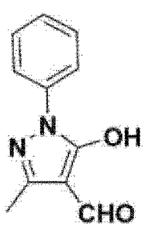
A4

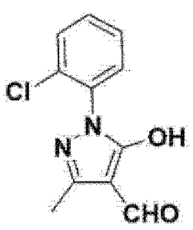
A5

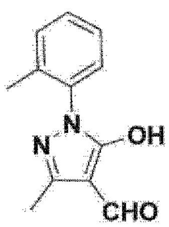
A6

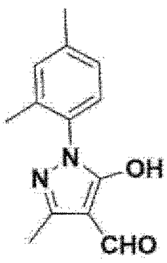
A7

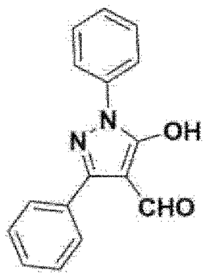
A8

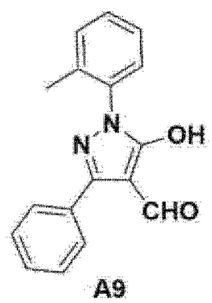
A9

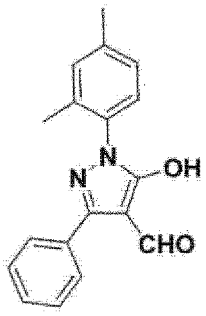
A10

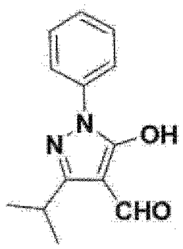
A11

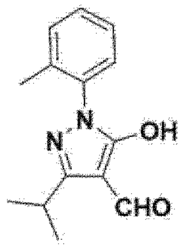
A12

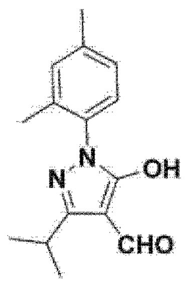
A13

(2) Synthesis of Intermediate B

[Reaction Scheme 2]

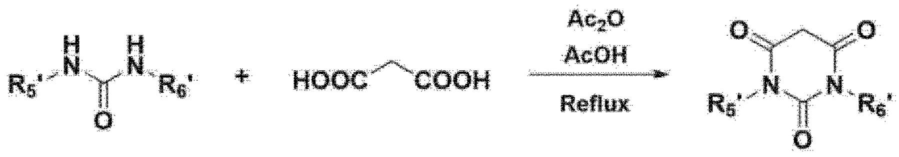


B1 B2 B

R5' = CH3, C2H5, C6H11, C6H5
R6' = CH3, C2H5, C6H11, C6H5

R5' = CH3, C2H5, C6H11, C6H5
R6' = CH3, C2H5, C6H11, C6H5

1 eq of Monomer B1, 1.5 eq of Monomer B2, and 2 eq of anhydrous acetic acid were diluted in 12 eq of acetic acid, and the resulting mixture was heated and stirred. After the reaction was completed, a solid was produced by adding water thereto, and the produced solid was filtered under reduced pressure while being washed using water and ethanol, thereby securing Intermediate B. The following Intermediates B3 to B6 were secured by changing the type of substituent to be introduced into Intermediate B.

Synthesis Example 1. Synthesis of Compound P1

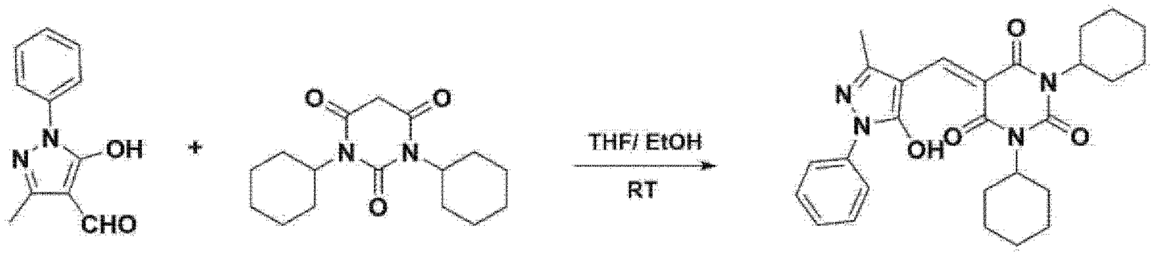


A4 B3 P1

Compound A4 (1 g, 1 eq) synthesized by the method of the above Reaction Scheme 1 and Compound B3 (1.16 g, 0.8 eq) synthesized by the method of Reaction Scheme 2 were diluted in 10 mL of tetrahydrofuran and 10 mL of ethanol, and the resulting mixture was stirred at room temperature under nitrogen. After the reaction was completed, the solvent was condensed, and extraction was performed using chloroform and water. After water was removed from the organic layer secured by extraction using anhydrous magnesium sulfate, the solvent was concentrated by distillation under reduced pressure. The concentrated product was recrystallized using chloroform and acetonitrile to secure Compound P1. (1.7 g, yield 72%) HR LC/MS/MS m/z calcd for $C_{27}H_{32}N_4O_4$ (M+) : 476.2424; found: 476.2424

**Synthesis Example 2. Synthesis of Compound P2**

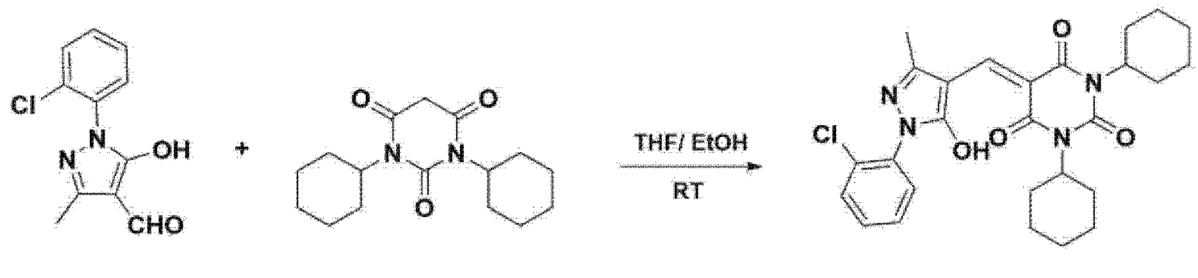


A5 B3 P2

Compound P2 was secured by performing the reaction in the same manner as in Synthesis Example 1, except that A5 (0.5 g, 1 eq) was used instead of A4 used in Synthesis Example 1. (0.87 g, yield 87%) HR LC/MS/MS m/z calcd for $C_{27}H_{31}ClN_4O_4$ (M+) : 510.2034; found: 510.2033

**Synthesis Example 3. Synthesis of Compound P3**

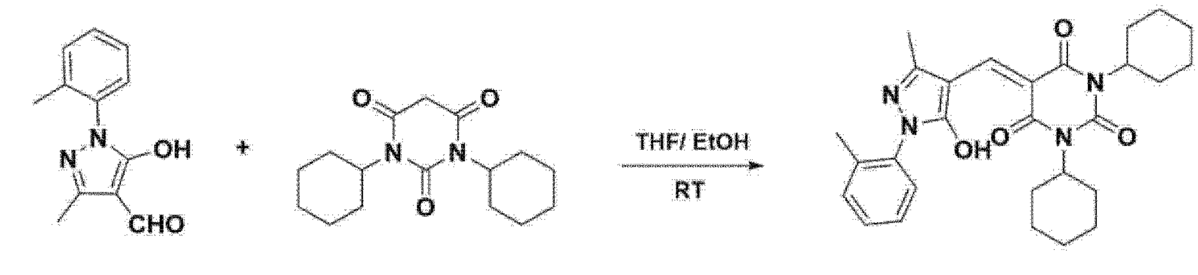


A6 B3 P3

Compound P3 was secured by performing the reaction in the same manner as in Synthesis Example 1, except that A6 (1.5 g, 1 eq) was used instead of A4 used in Synthesis Example 1. (2.62 g, yield 77%) HR LC/MS/MS m/z calcd for $C_{28}H_{34}N_4O_4$ (M+) : 490.2580; found: 490.2581

Synthesis Example 4. Synthesis of Compound P4

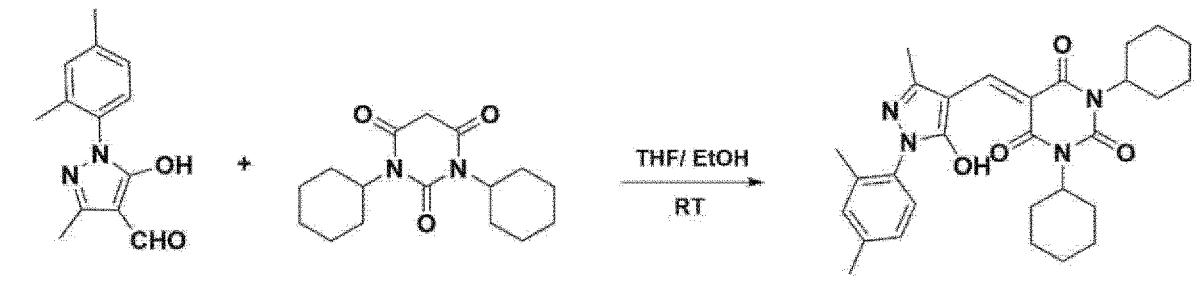


A7 B3 P4

Compound P4 was secured by performing the reaction in the same manner as in Synthesis Example 1, except that A7 (1.0 g, 1 eq) was used instead of A4 used in Synthesis Example 1. (1.18 g, yield 54%) HR LC/MS/MS m/z calcd for $C_{29}H_{36}N_4O_4$ (M+) : 504.2737; found: 504.2736

Synthesis Example 5. Synthesis of Compound P5

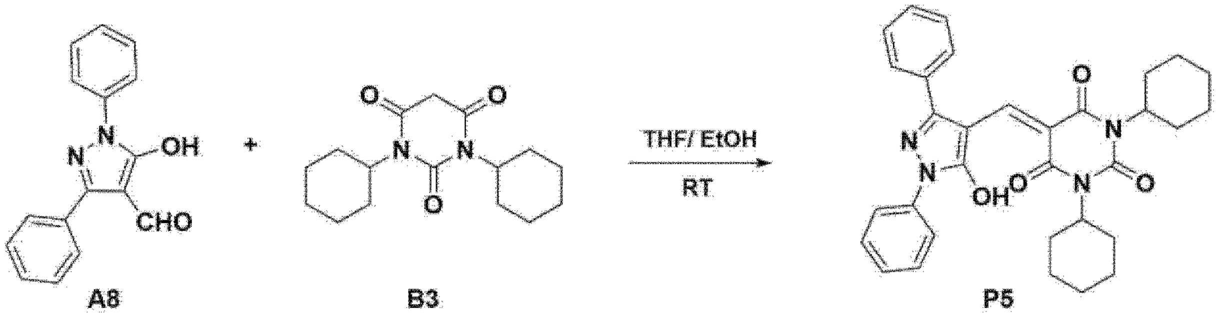


A8 B3 P5

Compound P5 was secured by performing the reaction in the same manner as in Synthesis Example 1, except that A8 (0.5 g, 1 eq) was used instead of A4 used in Synthesis Example 1. (1.18 g, yield 54%) HR LC/MS/MS m/z calcd for $C_{32}H_{34}N_4O_4$ (M+) : 538.2580; found: 538.2580

**Synthesis Example 6. Synthesis of Compound P6**

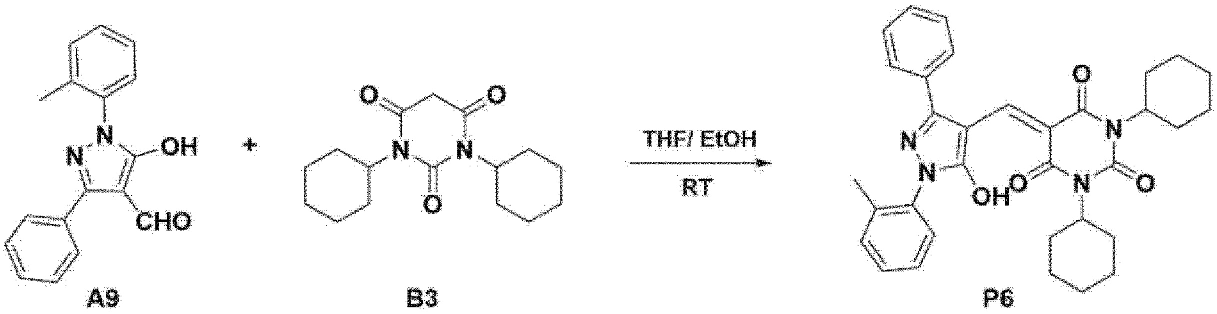


A9 B3 P6

Compound P6 was secured by performing the reaction in the same manner as in Synthesis Example 1, except that A9 (0.7 g, 1 eq) was used instead of A4 used in Synthesis Example 1. (1.04 g, yield 75%) HR LC/MS/MS m/z calcd for $C_{33}H_{36}N_4O_4$ (M+) : 552.2737; found: 552.2736

**Synthesis Example 7. Synthesis of Compound P7**

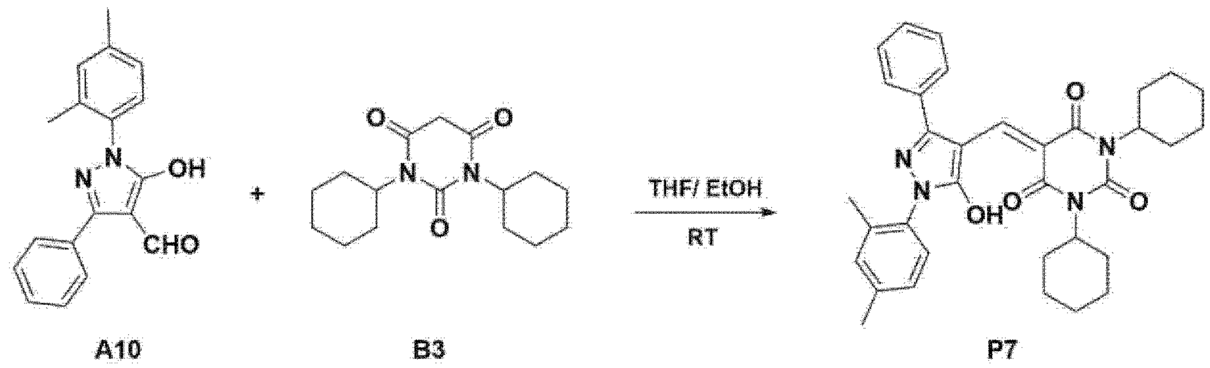


A10 B3 P7

Compound P7 was secured by performing the reaction in the same manner as in Synthesis Example 1, except

that A10 (1.0 g, 1 eq) was used instead of A4 used in Synthesis Example 1. (1.34 g, yield 69%) HR LC/MS/MS m/z calcd for $C_{34}H_{38}N_4O_4$ (M+) : 566.2893; found: 566.2893

**Synthesis Example 8. Synthesis of Compound P8**

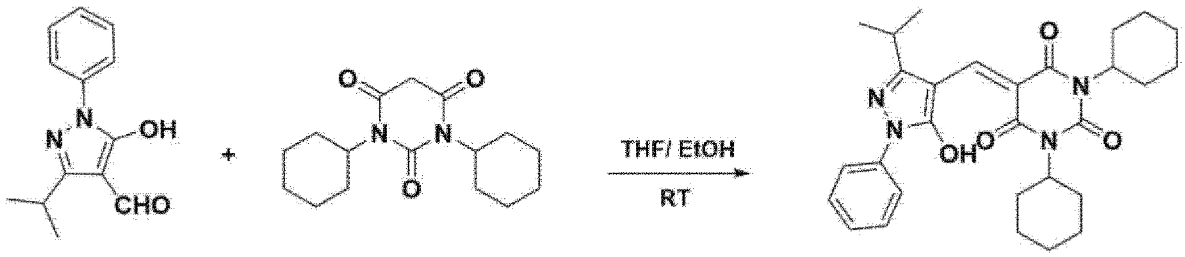


A11 B3 P8

Compound P8 was secured by performing the reaction in the same manner as in Synthesis Example 1, except that A11 (1.0 g, 1 eq) was used instead of A4 used in Synthesis Example 1. (1.78 g, yield 81%) HR LC/MS/MS m/z calcd for $C_{29}H_{36}N_4O_4$ (M+) : 504.2737; found: 504.2737

**Synthesis Example 9. Synthesis of Compound P9**

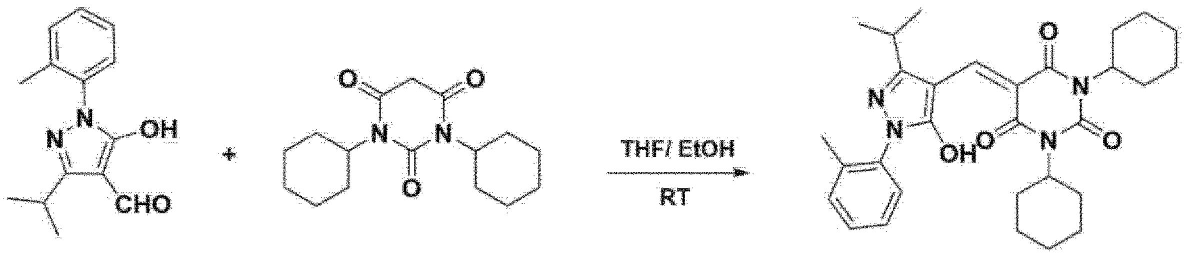


A12 B3 P9

Compound P9 was secured by performing the reaction in the same manner as in Synthesis Example 1, except that A12 (1.0 g, 1 eq) was used instead of A4 used in Synthesis Example 1. (1.51 g, yield 71%) HR LC/MS/MS m/z calcd for $C_{30}H_{38}N_4O_4$ (M+) : 518.2893; found: 518.2893

**Synthesis Example 10. Synthesis of Compound P10**

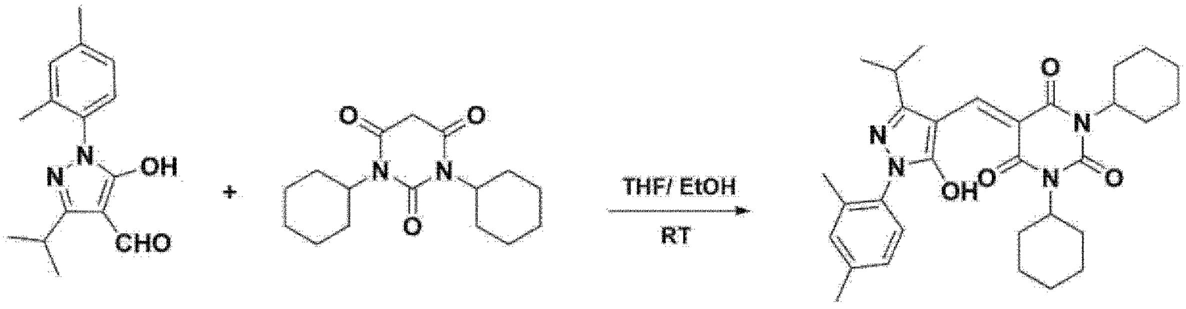


A13 B3 P10

Compound P10 was secured by performing the reaction in the same manner as in Synthesis Example 1, except that A13 (1.2 g, 1 eq) was used instead of A4 used in Synthesis Example 1. (1.56 g, yield 63%) HR LC/MS/MS m/z calcd for $C_{31}H_{40}N_4O_4$ (M+) : 532.3050; found: 532.3051

**Synthesis Example 11. Synthesis of Compound P11**

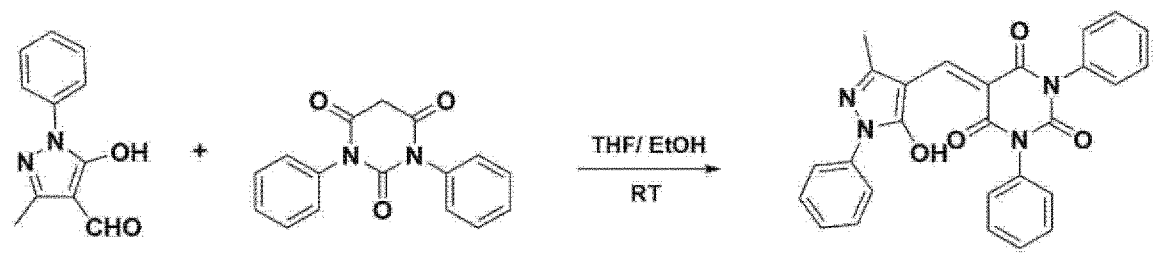


A4 B4 P11

Compound P11 was secured by performing the reaction in the same manner as in Synthesis Example 1, except that A4 (1.0 g, 1 eq) and B4 (1.11 g, 0.8 eq) instead of B3 were used likewise in Synthesis Example 1. (1.95 g, yield 85%) HR LC/MS/MS m/z calcd for $C_{27}H_{20}N_4O_4$ (M+) : 464.1485; found: 464.1486

**Synthesis Example 12. Synthesis of Compound P12**

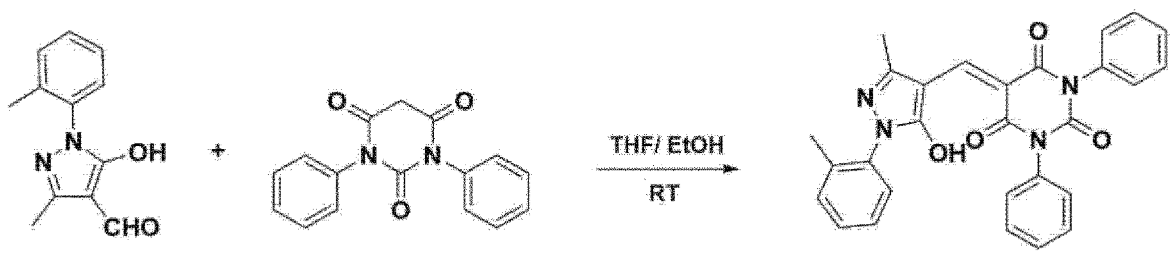


A6 B4 P12

Compound P12 was secured by performing the reaction in the same manner as in Synthesis Example 1, except that A6 (1.5 g, 1 eq) and B4 (1.56 g, 0.8 eq) instead of A4 and B3, respectively, were used likewise in Synthesis Example 1. (1.79 g, yield 54%) HR LC/MS/MS m/z calcd for $C_{28}H_{22}N_4O_4$ (M+) : 478.1641; found: 478.16421

## Synthesis Example 13. Synthesis of Compound P13

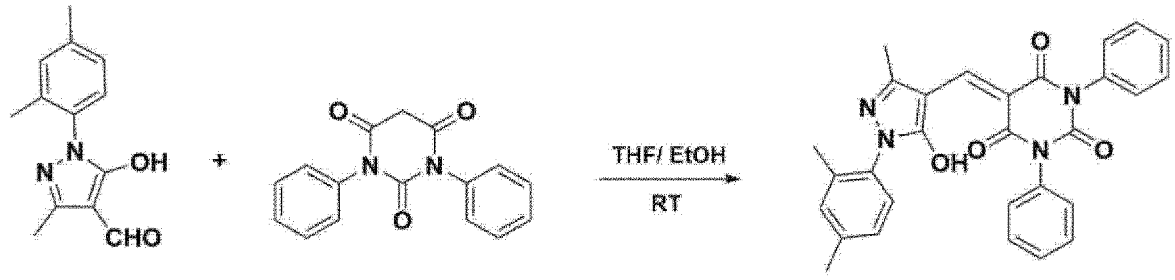


A7 B4 P13

Compound P13 was secured by performing the reaction in the same manner as in Synthesis Example 1, except that A7 (1.5 g, 1 eq) and B4 (1.46 g, 0.8 eq) instead of A4 and B3, respectively, were used likewise in Synthesis Example 1. (1.64 g, yield 51%) HR LC/MS/MS m/z calcd for $C_{29}H_{24}N_4O_4$ (M+) : 492.1798; found: 492.1798

## Synthesis Example 14. Synthesis of Compound P14

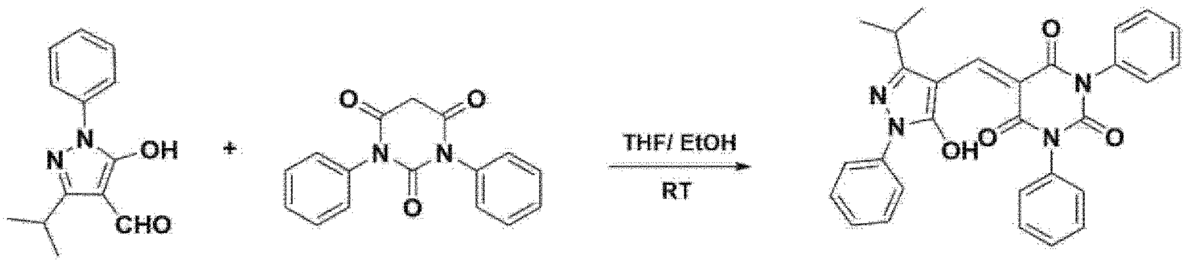


A11 B4 P14

Compound P14 was secured by performing the reaction in the same manner as in Synthesis Example 1, except that A11 (1.0 g, 1 eq) and B4 (0.97 g, 0.8 eq) instead of A4 and B3, respectively, were used likewise in Synthesis Example 1. (1.43 g, yield 67%) HR LC/MS/MS m/z calcd for $C_{29}H_{24}N_4O_5$ (M+) : 492.1798; found: 492.1797

## Synthesis Example 15. Synthesis of Compound P15

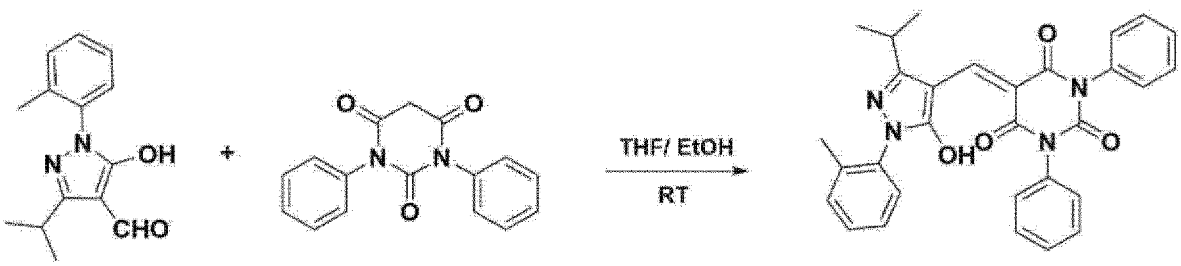


A12 B4 P15

Compound P15 was secured by performing the reaction in the same manner as in Synthesis Example 1, except that A12 (1.3 g, 1 eq) and B4 (1.19 g, 0.8 eq) instead of A4 and B3, respectively, were used likewise in Synthesis Example

1. (1.89 g, yield 70%) HR LC/MS/MS m/z calcd for $C_{30}H_{26}N_4O_4$ (M+) : 506.1954; found: 506.1954

**Synthesis Example 16. Synthesis of Comparative Compound Z1**

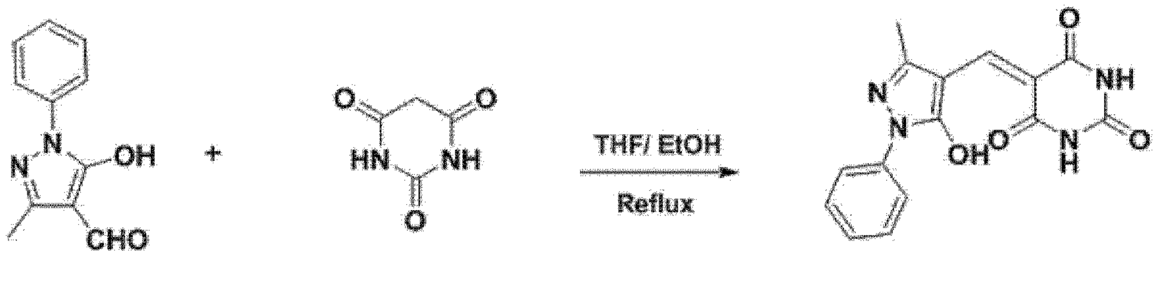


A4 B5 Z1

Comparative Compound Z1 was secured by performing a heating reaction in the same manner as in Synthesis Example 1, except that A4 (2.0 g, 1 eq) and B5 (1.01 g, 0.8 eq) instead of B3 were used likewise in Synthesis Example 1. (0.99 g, yield 32 %) HR LC/MS/MS m/z calcd for $C_{15}H_{12}N_4O_4$ (M+): 312.0895; found: 312.0896

**Synthesis Example 17. Synthesis of Comparative Compound Z2**

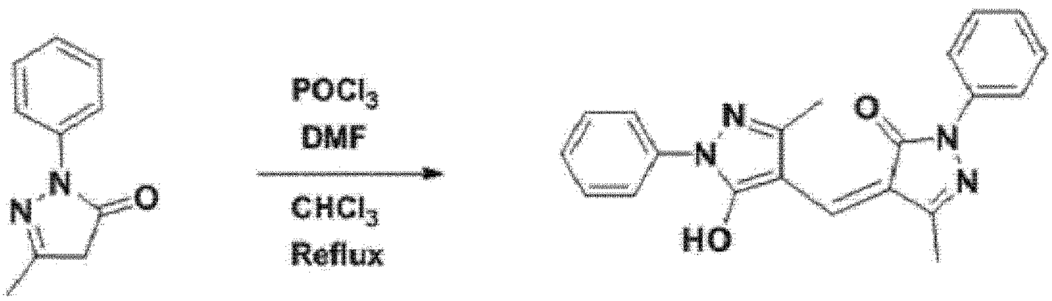


A3 Z2

After stirring was performed for 30 minutes by putting 5 eq of dimethylformamide into the chloroform solvent while maintaining the temperature at 0°C under nitrogen, 2 eq of $POC_3$ was slowly added dropwise thereto. After the addition of $POCl_3$ was completed, the temperature was maintained at low temperature for 30 minutes, and then the mixture was stirred at room temperature for 30 minutes by adding 1 eq of Monomer A3 thereto, and heated. After the reaction was completed, extraction was performed using an aqueous solution of chloroform and potassium acetate (KOAc), and moisture was removed by adding anhydrous magnesium sulfate to the extracted organic solvent layer. The organic layer from which moisture was removed was condensed to secure Comparative Compound Z2 using ethanol. (3.21 g, yield 32 %) HR LC/MS/MS m/z calcd for $C_{21}H_{18}N_4O_2$ (M+): 354.1328; found: 354.1328

**<Experimental Examples>**

**Example 1.**

Based on 100 parts by weight a solid content except for a solvent in an adhesive composition, 95.4 parts by weight of an adhesive material (AD-701 solid content, LG Chem.), 0.29 part by weight of Compound P1, 0.1 part by weight of an isocyanate-based cross-linking agent (T39M, Soken Chemical & Engineering Co., Ltd.), 0.2 part by weight of a silane-based coupling agent (T-789J, Soken Chemical & Engineering Co., Ltd.), 0.5 part by weight of an antioxidant

(Kinox-80, Hannong Chemicals Inc.), 1.5 parts by weight of an antistatic agent (FC-4400, 3M), 2 parts by weight of a hindered amine light stabilizer (Tinuvin 123, BASF SE), and 0.001 part by weight of a catalyst (dibutyltin dilaurate, Sigma-Aldrich) were added to the adhesive composition, the solvent (methyl ethyl ketone, MEK) was added thereto in an amount of 25 parts by weight based on the total parts by weight of the adhesive composition, and a release layer (PET) was coated with the adhesive composition mixed using a mixer (Shaker, SKC 6100, JEIO Tech.) to have a thickness of 22 um to 23 um using a knife bar coating apparatus (KP-3000, Kipae E&T Co., Ltd.), thereby preparing an adhesive film. After coating, the release layer was removed, and the adhesive film and a binder resin film (TAC: cellulose triacetate) were sequentially laminated on glass through lamination, thereby producing a sample. The binder resin film (TAC: cellulose triacetate) was used to produce a sample using an adhesive film including an adhesive composition including Compound P1, and does not affect the measured physical properties of the adhesive film.

The maximum absorption wavelength λmax (nm) of each of the adhesive composition and the adhesive film was measured using a UV-vis apparatus (Shimazu UV-3600). In addition, the transmittance of the sample for light with a wavelength of 390 to 450 nm was measured by the UV-vis apparatus (Shimazu UV-3600). Under conditions of light fastness 55,600 lx * 48 hours; under conditions of heat resistance 80°C and for 500 hours; or under conditions of moisture resistance 60°C, 90% RH and for 500 hours, the sample was each exposed, and then the transmittance for light with a wavelength of 390 to 450 nm was re-measured. ΔT was calculated by dividing the value obtained by subtracting the transmittance immediately after the sample was produced from the re-measured transmittance by the transmittance value immediately after the sample was produced, and the results under the evaluation conditions are shown in Table 1. Furthermore, the transmittance of the sample for light in a region with a wavelength of 450 nm or more was measured by the UV-vis apparatus (Shimazu UV-3600). Further, the solubility of Compound p1 to methyl ethyl ketone (MEK) was measured under conditions of 1 atm and room temperature.

ΔT(%)={(transmittance measured immediately after the optical film was prepared - transmittance re-measured after the optical film is exposed to each condition of light fastness, heat resistance, and moisture resistance)/transmittance measured immediately after the optical film is prepared}X100

**Examples 2 to 10 and Comparative Examples 1 and 2**

Experiments were performed under the same conditions as in Example 1, except that Compounds P3 to P10, Compound P15 and Comparative Compounds Z1 and Z2 were used instead of Compound P1, respectively, in Example 1, and the experimental results are shown in the following Table 1.

[Table 1]

| | Compound | λmax (nm) (Solution) | λmax (nm) (Thin film) | Light fastness | Heat resistance | Moisture resistance | MEK solubility | Wavelength compatibility |
|---|---|---|---|---|---|---|---|---|
| Example 1 | P1 | 411 | 407 | △ | △ | ○ | △ | ○ |
| Example 2 | P3 | 404 | 400 | ○ | ○ | ○ | ○ | ○ |
| Example 3 | P4 | 403 | 399 | ○ | ○ | ○ | ○ | ○ |
| Example 4 | P5 | 407 | 406 | | ○ | ○ | ○ | ○ |
| Example 5 | P6 | 400 | 400 | ○ | △ | ○ | ○ | ○ |
| Example 6 | P7 | 399 | 399 | ○ | ○ | ○ | ○ | ○ |
| Example 7 | P8 | 406 | 408 | ○ | △ | ○ | △ | ○ |
| Example 8 | P9 | 403 | 399 | ○ | ○ | ○ | ○ | ○ |
| Example 9 | P10 | 403 | 398 | ○ | ○ | ○ | ○ | ○ |
| Example 10 | P15 | 402 | 398 | ○ | ○ | ○ | △ | ○ |
| Comparative Example 1 | Z1 | 412 | 425 | x | x | △ | x | △ |
| Comparative Example 2 | Z2 | 405 | 403 | ○ | ○ | ○ | ○ | x |

[Evaluation of light fastness, heat resistance, and moisture resistance]
O: ΔT 1.5% or less
△: ΔT more than 1.5% and 3% or less
X: ΔT more than 3%

The light fastness, heat resistance and moisture resistance were evaluated as O, △, and X based on whether the ΔT was 1.5% or less, more than 1.5% and 3% or less, or more than 3%, respectively.

[Evaluation of MEK solubility]

O: 0.3 wt.% or more
△: more than 0.1 wt.% and less than 0.3 wt.%
X: 0.1 wt.% or less

The solubility was evaluated as O, △, and X based on whether the solubility of each compound to 10 g of methyl ethyl ketone was 0.3 wt.% or more, more than 0.1 wt.% and less than 0.3 wt.%, or 0.1 wt.% or less, respectively.

[Evaluation of wavelength compatibility]

O: transmittance of 90% or more in region with 450 nm or more
△: transmittance of 80% or more in region with 450 nm or more
X: transmittance of 80% or less in region with 450 nm or more

The wavelength compatibility was evaluated as O, △, and X based on whether the transmittance in a region with 450 nm or more was 90% or more, 80% or more, or 80% or less, respectively.

From the experimental results in Table 1, it can be confirmed that the adhesive film including the compound of Chemical Formula H according to an exemplary embodiment of the present invention as described below is excellent in light fastness, heat resistance, moisture resistance, solubility to solvent and/or wavelength compatibility compared to other films. As in the present invention, an effect as an optical film can be obtained only when the adhesive composition or adhesive film included in forming the optical film needs to be excellent in all of the light fastness, heat resistance, moisture resistance, solubility to solvent, and wavelength compatibility, but in the compounds used in Examples 1 to 10, a pyrazole derivative is linked to a hetero ring including two or more N's via propylene according to Chemical Formula H of the present application and a cycloalkyl group or an aryl group is introduced into the N, so that the compounds have a resonance structure and a molecular structure arrangement capable of hydrogen bonding, and as a result, it can be confirmed that the compounds used in Examples 1 to 10 are excellent in all of the light fastness, heat resistance, moisture resistance, solubility to solvent, and wavelength compatibility compared to the Comparative Examples.

When Examples 1 to 10 are compared to Comparative Examples 1 and 2, a cycloalkyl group is not introduced into the positions corresponding to R3 and R4 of Chemical Formula H of the present application in Comparative Compound z1 of Comparative Example 1, so that it can be confirmed that at least one of the light fastness, heat resistance, moisture resistance, solubility to solvent, and wavelength compatibility remarkably deteriorates compared to Examples 1 to 10.

When Examples 1 to 10 are compared to Comparative Examples 1 and 2, a pyrazole derivative is not introduced into Comparative Compound Z2 in Comparative Example 2, so that it can be confirmed that the wavelength compatibility remarkably deteriorates compared to Examples 1 to 10. As in the content of the UV-Vis spectra illustrated in FIG. 2, Compound P3 of the present invention has a wavelength distribution narrower and more optimized than Comparative Compound Z2 used in Comparative Example 2, so that it can be confirmed that the wavelength compatibility is excellent.

## Claims

1. A compound represented by the following Chemical Formula H:

[Chemical Formula H]

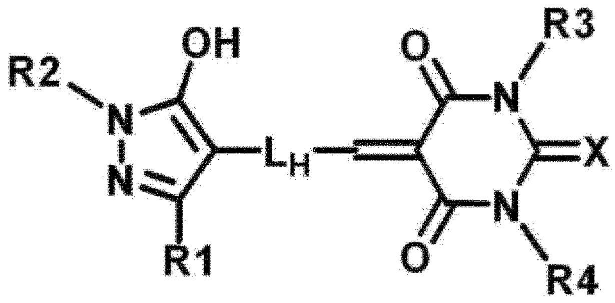

in Chemical Formula H,

X is O or S,
$L_H$ is a direct bond; or a substituted or unsubstituted alkylene group,
R1 is -C(=O)ORa; a substituted or unsubstituted alkyl group; a substituted or unsubstituted cycloalkyl group; a substituted or unsubstituted aryl group; or a substituted or unsubstituted heterocyclic group,
R2 is -C(=O)Rb; a substituted or unsubstituted alkyl group; a substituted or unsubstituted cycloalkyl group; a substituted or unsubstituted aryl group; or a substituted or unsubstituted heterocyclic group,
Ra is hydrogen; deuterium; or a substituted or unsubstituted alkyl group,
Rb is hydrogen; deuterium; a hydroxyl group; a substituted or unsubstituted alkyl group; a substituted or unsubstituted aryl group; or a substituted or unsubstituted heterocyclic group, and
R3 and R4 are the same as or different from each other, and are each independently a substituted or unsubstituted cycloalkyl group; or a substituted or unsubstituted aryl group.

**2.** The compound of claim 1, wherein Chemical Formula H is represented by the following Chemical Formula H-1:

[Chemical Formula H-1]

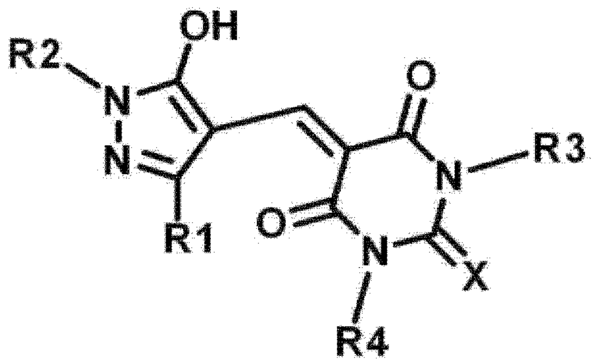

in Chemical Formula H-1,
X and R1 to R4 are the same as those defined in Chemical Formula H.

**3.** The compound of claim 1, wherein R1 is an alkyl group; a cycloalkyl group; or an aryl group which is unsubstituted or substituted with one or two or more substituents selected from the group consisting of a halogen group and an alkyl group.

**4.** The compound of claim 1, wherein R2 is an alkyl group; a cycloalkyl group; or an aryl group which is unsubstituted or substituted with one or two or more substituents selected from the group consisting of a chloro group, a fluoro group, $-OCH_3$ and a methyl group.

**5.** The compound of claim 1, wherein R3 and R4 are the same as or different from each other, and are each independently a substituted or unsubstituted cycloalkyl group having 3 to 10 carbon atoms; or a substituted or unsubstituted aryl group having 6 to 10 carbon atoms.

**6.** The compound of claim 1, wherein R3 and R4 are the same as or different from each other, and are each independently any one selected from the group consisting of the following structures:

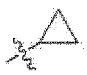 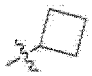 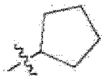 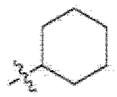 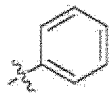

in the structures,

the structures are unsubstituted or substituted with deuterium; a halogen group; -ORe'; or a substituted or unsubstituted alkyl group,
Re' is hydrogen; deuterium; or a substituted or unsubstituted alkyl group, and

" 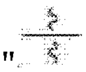 "

means a site linked to Chemical Formula H.

**7.** The compound of claim 1, wherein Chemical Formula H is any one selected from the group consisting of the following compounds:

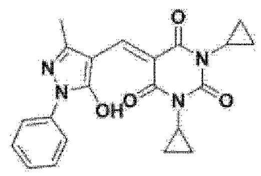 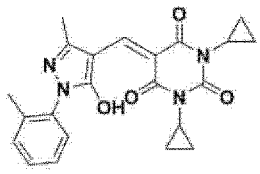 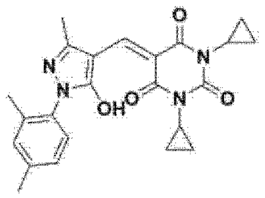 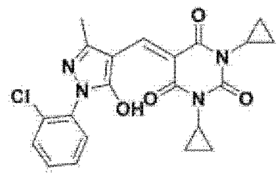

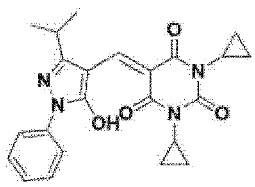 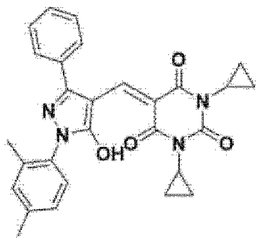 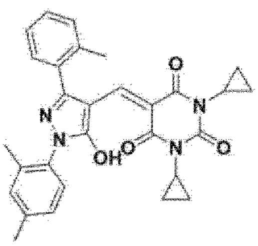 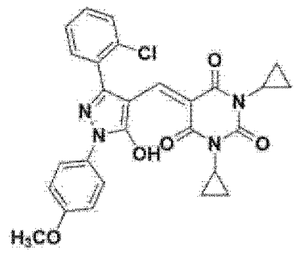

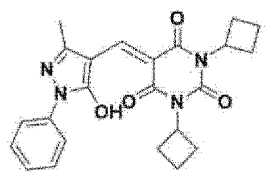 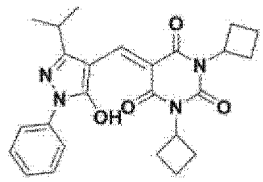 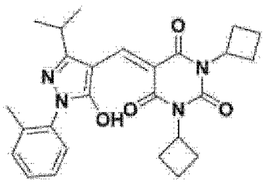 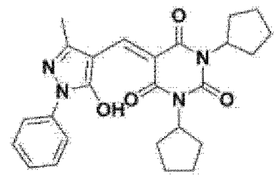

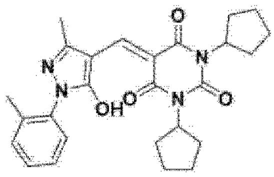 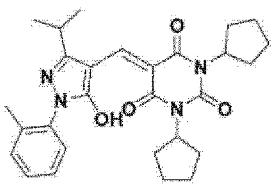 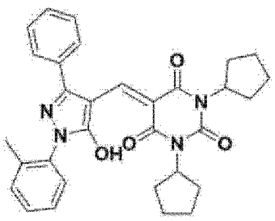 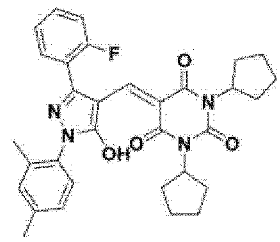

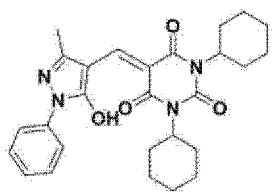
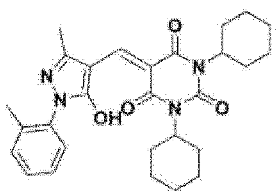
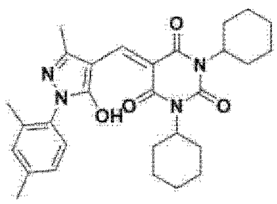
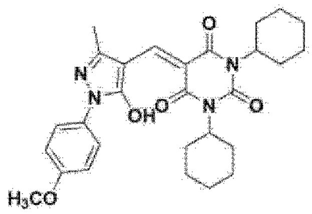

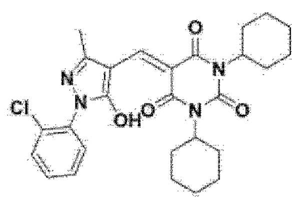
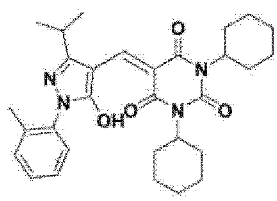
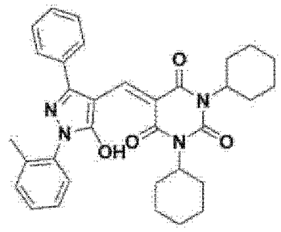
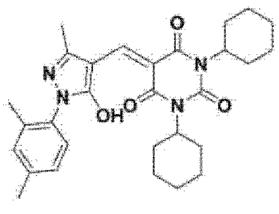

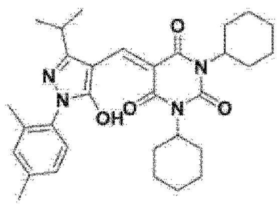
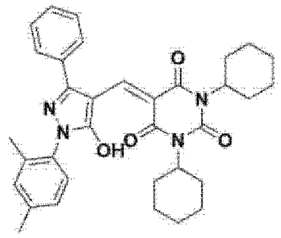
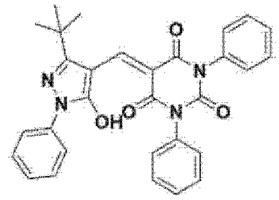
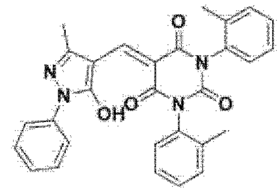

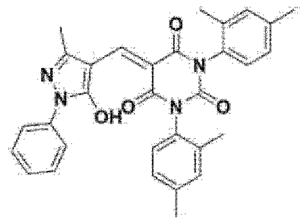
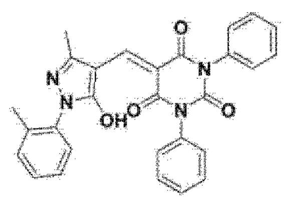
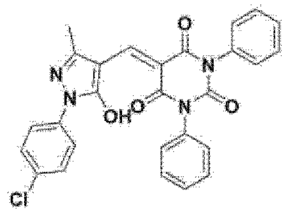
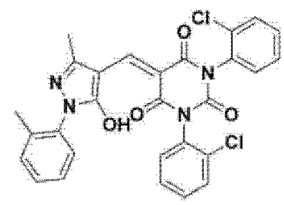

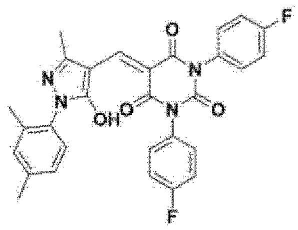
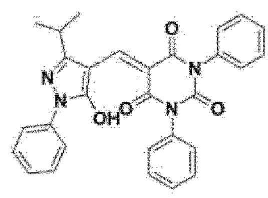
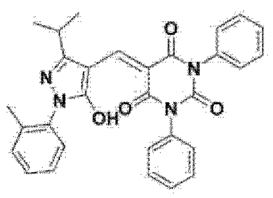
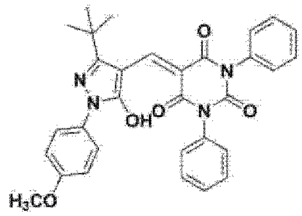

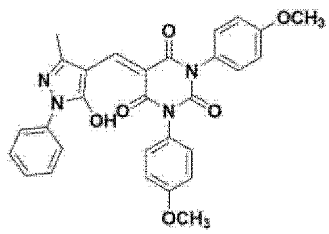
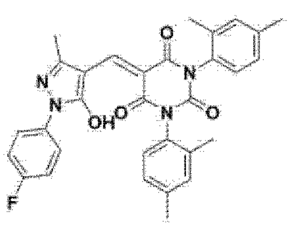

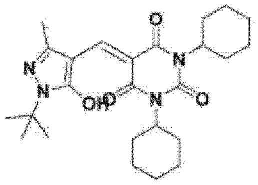 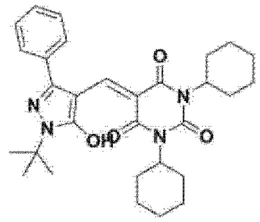 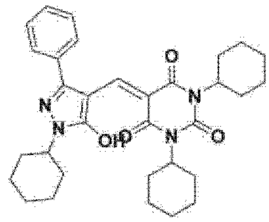 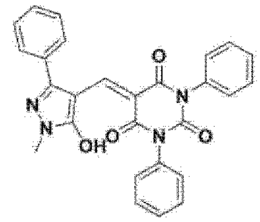

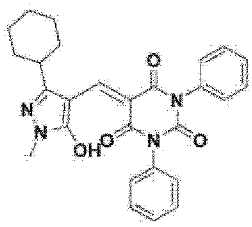 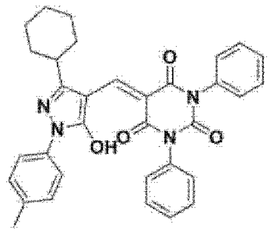 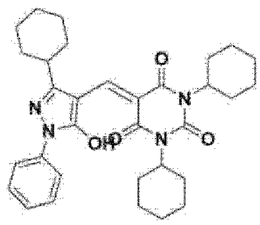

**8.** An adhesive composition comprising: an adhesive material; and the compound according to any one of claims 1 to 7.

**9.** The adhesive composition of claim 8, wherein the adhesive material is included in an amount of 93 parts by weight to 97 parts by weight based on 100 parts by weight of a solid content of the adhesive composition.

**10.** The adhesive composition of claim 8, wherein the compound is included in an amount of 0.1 part by weight to 3 parts by weight based on 100 parts by weight of a solid content of the adhesive composition.

**11.** The adhesive composition of claim 8, further comprising one or more selected from the group consisting of a cross-linking agent, a silane-based coupling agent, an antioxidant, an antistatic agent, a light stabilizer and a catalyst.

**12.** The adhesive composition of claim 8, further comprising a cross-linking agent, a silane-based coupling agent, an antioxidant, an antistatic agent, a light stabilizer and a catalyst,

wherein based on 100 parts by weight of a solid content of the adhesive composition,
a content of the cross-linking agent is 0.05 part by weight to 0.2 part by weight, a content of the silane-based coupling agent is 0.1 part by weight to 0.4 part by weight, a content of the antioxidant is 0.2 part by weight to 0.8 part by weight, a content of the antistatic agent is 0.5 part by weight to 2.5 parts by weight, a content of the light stabilizer is 1 part by weight to 4 parts by weight, and a content of the catalyst is 0.005 part by weight to 0.02 part by weight.

**13.** An adhesive film comprising the adhesive composition according to claim 8.

**14.** The adhesive film of claim 13, wherein under conditions of light fastness 55,600 lx * 48 hours; conditions of heat resistance 80°C and 500 hours; or conditions of moisture resistance 60°C, 90% RH and 500 hours,
a change in transmittance for light with a wavelength of 390 to 450 nm is each 3% or less.

**15.** An electronic device comprising the adhesive film according to claim 13.

[Figure 1]

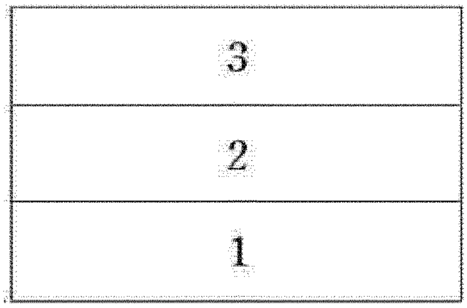


[Figure 2]

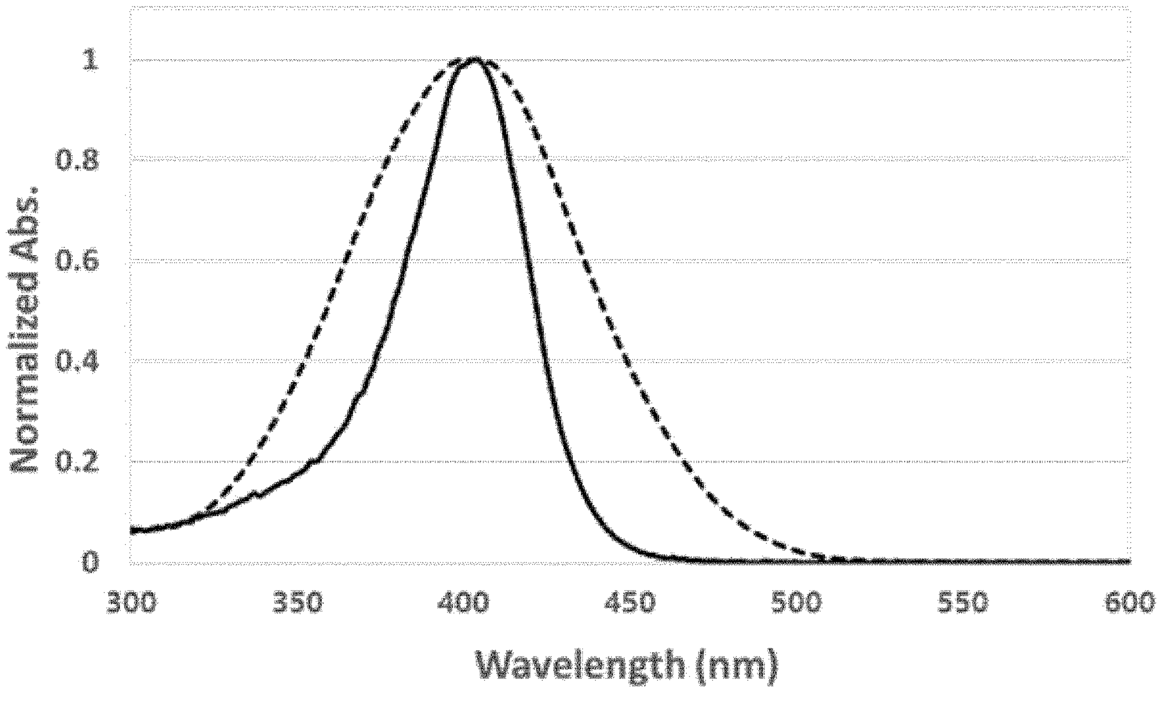

**INTERNATIONAL SEARCH REPORT**

International application No.
**PCT/KR2022/001436**

**A. CLASSIFICATION OF SUBJECT MATTER**

**C09J 11/06**(2006.01)i; **C08K 5/3445**(2006.01)i; **C08K 5/3462**(2006.01)i; **C08K 5/45**(2006.01)i; **C09J 7/22**(2018.01)i

According to International Patent Classification (IPC) or to both national classification and IPC

**B. FIELDS SEARCHED**

Minimum documentation searched (classification system followed by classification symbols)

C09J 11/06(2006.01); C08L 63/00(2006.01); G02B 5/22(2006.01); G02B 5/30(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 접착 필름(adhesive film), 피라졸(pyrazole), 피리미딘트리온(pyrimidinetrione), 티옥소-피리미딘디온(thioxo-pyrimidinedione)

**C. DOCUMENTS CONSIDERED TO BE RELEVANT**

| Category* | Citation of document, with indication, where appropriate, of the relevant passages | Relevant to claim No. |
|---|---|---|
| X<br>A | Chemical Abstract Compound. STN Next RN 1210386-71-2 (16 March 2010).<br>See the compounds. | 1,2,4-6<br>3,7-15 |
| X | Chemical Abstract Compound. STN Next RN 1210214-85-9 (16 March 2010).<br>See the compounds. | 1,2,4-6 |
| X | Chemical Abstract Compound. STN Next RN 489408-28-8 (13 February 2003).<br>See the compounds. | 1,2,4-6 |
| X | Chemical Abstract Compound. STN Next RN 482279-85-6 (28 January 2003).<br>See the compounds. | 1,2,4-6 |
| X | Chemical Abstract Compound. STN Next RN 482279-73-2 (28 January 2003).<br>See the compounds. | 1,2,4-6 |
| X | Chemical Abstract Compound. STN Next RN 482279-53-8 (28 January 2003).<br>See the compounds. | 1,2,4-6 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **24 May 2022** | **24 May 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208**<br>Facsimile No. **+82-42-481-8578** | Telephone No. |

Form PCT/ISA/210 (second sheet) (July 2019)

**INTERNATIONAL SEARCH REPORT**

International application No.
**PCT/KR2022/001436**

**C. DOCUMENTS CONSIDERED TO BE RELEVANT**

| Category* | Citation of document, with indication, where appropriate, of the relevant passages | Relevant to claim No. |
|---|---|---|
| X | Chemical Abstract Compound. STN Next RN 482279-38-9 (28 January 2003).<br>See the compounds. | 1,2,4-6 |
| A | JP 09-100392 A (HITACHI CHEM CO., LTD.) 15 April 1997 (1997-04-15)<br>See abstract; claims 1 and 3; and paragraphs [0007] and [0008]. | 1-15 |
| A | US 3364211 A1 (STANLEY, L. N.) 16 January 1968 (1968-01-16)<br>See abstract; and claims 1-3. | 1-15 |
| A | JP 2017-142412 A (SUMITOMO CHEMICAL CO., LTD.) 17 August 2017 (2017-08-17)<br>See claims 7-9. | 1-15 |

Form PCT/ISA/210 (second sheet) (July 2019)

**INTERNATIONAL SEARCH REPORT**
**Information on patent family members**

International application No.
**PCT/KR2022/001436**

| Patent document cited in search report | Publication date (day/month/year) | Patent family member(s) | Publication date (day/month/year) |
|---|---|---|---|
| JP 09-100392 A | 15 April 1997 | JP 09-100392 A | 15 April 1997 |
| US 3364211 A1 | 16 January 1968 | None | |
| JP 2017-142412 A | 17 August 2017 | CN 107085257 A | 22 August 2017 |
| | | CN 107085257 B | 01 June 2021 |
| | | JP 6777401 B2 | 28 October 2020 |
| | | KR 10-2017-0095147 A | 22 August 2017 |
| | | TW 201741423 A | 01 December 2017 |
| | | US 2017-0235032 A1 | 17 August 2017 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210014649
- KR 1020210074736
- JP 2012211305 A